(19) Europäisches Patentamt
European Patent Office
Office européen des brevets

(11) EP 3 315 190 A1

(12) # EUROPEAN PATENT APPLICATION
published in accordance with Art. 153(4) EPC

(43) Date of publication:
**02.05.2018 Bulletin 2018/18**

(21) Application number: **16814412.9**

(22) Date of filing: **22.06.2016**

(51) Int Cl.:
***B01D 71/68*** (2006.01)
***A61K 35/14*** (2015.01)
***A61M 1/18*** (2006.01)
***A61P 7/08*** (2006.01)
***B01D 69/10*** (2006.01)
***B01D 69/12*** (2006.01)
***B01D 71/44*** (2006.01)
***C08J 7/04*** (2006.01)
***C08L 39/06*** (2006.01)
***C08L 81/06*** (2006.01)
***B01D 67/00*** (2006.01)
***B01D 69/02*** (2006.01)
***B01D 69/08*** (2006.01)
***B01D 71/40*** (2006.01)
***B01D 63/02*** (2006.01)

(86) International application number:
**PCT/JP2016/068571**

(87) International publication number:
**WO 2016/208642 (29.12.2016 Gazette 2016/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **23.06.2015 JP 2015125420**
**06.04.2016 JP 2016076397**

(71) Applicants:
- **Asahi Kasei Medical Co., Ltd.**
**Chiyoda-ku**
**Tokyo 101-8101 (JP)**
- **National University Corporation of Yamagata University**
**Yamagata-shi**
**Yamagata 990-8560 (JP)**

(72) Inventors:
- **MORITA, Naoki**
**Tokyo 101-8101 (JP)**
- **TERAJIMA, Shuji**
**Tokyo 101-8101 (JP)**
- **MIURA, Suguru**
**Tokyo 101-8101 (JP)**
- **INOUE, Satoru**
**Tokyo 101-8101 (JP)**
- **TANAKA, Masaru**
**Yonezawa-shi**
**Yamagata 992-8510 (JP)**

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(54) **SEPARATION MEMBRANE FOR BLOOD TREATMENT, AND BLOOD TREATMENT DEVICE INCORPORATING SEPARATION MEMBRANE**

(57) A separation membrane for blood processing, wherein the separation membrane for blood processing includes:
a separation membrane containing polysulfone-based polymer and polyvinylpyrrolidone; and
a coating film provided on at least a part of the surface of the separation membrane and containing a polymer material having a structure represented by the following general formula (1):

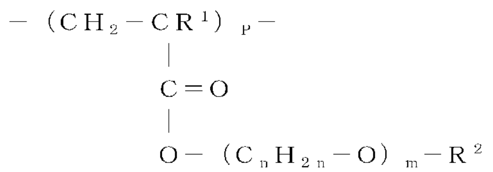
(1)

EP 3 315 190 A1

wherein $R^1$ is a hydrogen atom or a methyl group; $R^2$ is a methyl group or an ethyl group; n is 2 to 6 and m is 1 to 3; P denotes the number of repetition; and a plurality of each of $R^1$, $R^2$, n, and m present in one molecule may be the same or different.

Figure 1

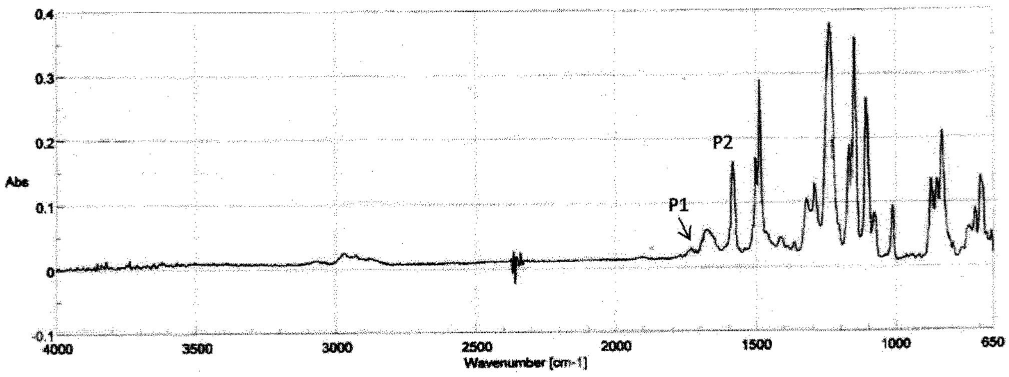

## Description

### Technical Field

The present invention relates to a separation membrane for blood processing to be used for separating and/or removing a particular substance in blood, and a blood processing device including the membrane.

### Background of Invention

In extracorporeal circulation therapies, hollow fiber membrane-type blood processing devices with a selective separation membrane are widely used. For example, hollow fiber membrane-type blood processing devices are used in hemodialysis as a maintenance therapy for a patient with chronic renal failure, continuous hemofiltration, continuous hemodiafiltration, or continuous hemodialysis as an acute blood purification therapy for a patient with a serious pathological condition such as acute renal failure and sepsis, and oxygenation to blood or plasmapheresis during a cardiotomy.

In these applications, it is required for a separation membrane to be excellent in mechanical strength and chemical stability, to allow easy control of permeation performance, and, in addition, to generate less eluted substance, to have less interaction with biological components, and to be safe for the living body.

In recent years, from the viewpoint of mechanical strength and chemical stability, and controllability for permeation performance, separation membranes consisting of polysulfone-based resin have been rapidly spreading. Since polysulfone-based resin is hydrophobic polymer, direct use of polysulfone-based resin results in a significantly insufficient hydrophilicity in the membrane surface and poor blood compatibility, and causes interactions with blood components to lead to frequent blood clotting, and in addition the permeation performance tends to degrade through adsorption of protein components and so on.

To compensate for the shortcomings, inclusion of hydrophilic polymer such as polyvinylpyrrolidone (PVP), polyvinyl alcohol, and polyethylene glycol, in addition to hydrophobic polymer such as polysulfone-based resin, for imparting blood compatibility has been examined. Known examples of methods for imparting blood compatibility include a method in which a membrane is formed by using a spinning dope containing hydrophobic polymer and hydrophilic polymer blended together and the membrane is dried to coat with hydrophilic polymer, and a method in which a membraned produced is brought into contact with a solution containing hydrophilic polymer and then dried to coat with hydrophilic polymer.

In extracorporeal circulation therapies, a blood processing device is used in a manner such that blood is directly contacted with a separation membrane in the blood processing device, and thus the separation membrane needs to be subjected to sterilization treatment before use.

For sterilization treatment, for example, ethylene oxide gas, high-pressure steam, or radiation has been used. However, ethylene oxide gas sterilization and high-pressure steam sterilization have problems including allergy caused by residual gas, the poor processing capability of sterilizers, and thermal deformation of materials, and thus radiation sterilization with $\gamma$-rays, electron beams, or the like is currently becoming the main stream.

While dry products are becoming the main stream for blood processing devices from the viewpoint of handleability and freezing during storage in a cold region, however, radiation sterilization in the presence of oxygen generates radicals, and the radicals generated bring about crosslinking reaction or decomposition of hydrophilic polymer, or furthermore causes oxidative degradation or the like thereof, which leads to denaturation of the membrane material, resulting in the degradation of the blood compatibility.

As a method for preventing degradation of a separation membrane due to such radioactive sterilization for products other than dry products, a method of filling a membrane module with an antioxidant solution followed by performing $\gamma$-ray sterilization to prevent oxidative degradation of the membrane (Patent Literature 1), and a method of filling with a pH buffer solution or an alkaline aqueous solution followed by sterilizing to prevent oxidation of the filling solution (Patent Literature 2) are disclosed.

For dry products, a method in which the oxygen concentration in sterilization is reduced to 0.001% or more and 0.1% or less (Patent Literature 3) is disclosed. In the technique according to Patent Literature 3, however, it is required, for example, to purge the inside of a packaging bag with an inert gas and then sterilize, or to charge a deoxidant in a packaging bag and sterilize after a certain period. Thus, a technique to fundamentally solve the problem of degradation of a hydrophilic polymer-containing separation membrane due to radiation sterilization in a dry state in the atmosphere has not been established yet.

Meanwhile, coating a hollow fiber separation membrane with blood-compatible polymer has been proposed to improve the blood compatibility.

However, there are problems including inhibition of the function of a hollow fiber separation membrane, for example, due to clogging of the micropores, depending on the type of polymer constituting the separation membrane, and, in some coating conditions, the thickness of a coating.

For example, Patent Literatures 4 and 5 each describe a hollow fiber membrane consisting of polypropylene, the surface of the hollow fiber membrane coated with PMEA (poly(2-methoxyethyl acrylate)) under specific conditions. However, uneven coating causes the variation of the blood compatibility, and thus a membrane having high blood compatibility is not necessarily obtained stably. For this reason, a membrane uniformly having high blood compatibility, without uneven coating, has been demanded in the field of blood processing.
Moreover, Patent Literature 6 discloses a polymer material having s structure similar to a structure represented by a general formula (1) in the present specification.
However, any of these literatures discloses neither a separation membrane containing polysulfone-based polymer and polyvinylpyrrolidone, nor sterilization treatment. As a natural consequence, no description is made on degradation of a separation membrane containing polysulfone-based polymer and polyvinylpyrrolidone and degradation of the blood compatibility in performing sterilization treatment for the separation membrane.

Citation List

Patent Literature

Patent Literature 1: Japanese Patent Laid-Open No. 4-338223
Patent Literature 2: Japanese Patent Laid-Open No. 7-194949
Patent Literature 3: International Publication No. WO 2006/016575
Patent Literature 4: Japanese Patent No. 3908839
Patent Literature 5: Japanese Patent Laid-Open No. 2015-136383
Patent Literature 6: Japanese Patent No. 4746984

Summary of Invention

Technical Problem

An object of the present invention is to provide a separation membrane for blood processing which is excellent in separation function and blood compatibility, with less degradation of the blood compatibility even after being subjected to radiation sterilization in a dry state in the atmosphere, and with no degradation of the blood compatibility even after a long-term use, and a blood processing device including the membrane, in particular, to realize such a separation membrane for blood processing with a substrate (separation membrane) coated with blood-compatible polymer.

Solution to Problem

The present inventors diligently examined to solve the above problems, and have found that one of the reasons why the blood compatibility of conventional separation membranes for blood processing coated with blood-compatible polymer is insufficient is presumed that adhesion between the hollow fiber membrane and the blood-compatible polymer coating is not good and a part of the layer coated on the separation membrane is not fixed because of uneven coating.
The present inventors further have found that a separation membrane for blood processing in which a separation membrane at least containing polysulfone-based polymer and polyvinylpyrrolidone is coated with a layer containing a polymer material having a structure represented by the following general formula (1) has highly excellent blood compatibility, which is maintained even after being subjected to sterilization in the atmosphere, and further has good adhesion between the separation membrane and the coated layer, causing no peeling of the coated layer and less degradation of the blood compatibility in use of the membrane, and thus completed the present invention.

$$-(CH_2-CR^1)_P- \quad \text{with side chain} \quad -C(=O)-O-(C_nH_{2n}-O)_m-R^2 \qquad (1)$$

In the formula, $R^1$ is a hydrogen atom or a methyl group; $R^2$ is a methyl group or an ethyl group; n is 2 to 6 and m is 1 to 3; P denotes the number of repetition; and a plurality of each of $R^1$, $R^2$, n, and m present in one molecule may be the same or different.

Specifically, the present invention is as follows.

[1] A separation membrane for blood processing, wherein the separation membrane for blood processing comprises:

a separation membrane containing polysulfone-based polymer and polyvinylpyrrolidone; and
a coating film provided on at least a part of the surface of the separation membrane and containing a polymer material having a structure represented by following general formula (1):

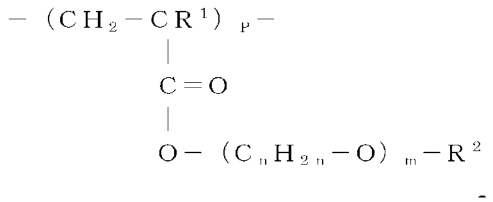

(1)

wherein $R^1$ is a hydrogen atom or a methyl group; $R^2$ is a methyl group or an ethyl group; n is 2 to 6 and m is 1 to 3; P denotes a number of repetition; and a plurality of each of $R^1$, $R^2$, n, and m present in one molecule is the same or different.

[2] The separation membrane for blood processing according to [1], wherein a number-average molecular weight of the polymer material having the structure represented by the general formula (1) is 8,000 to 300,000.
[3] The separation membrane for blood processing according to [1] or [2], wherein, in an infrared absorption curve obtained in attenuated total reflection-infrared spectroscopy (ATR-IR) for the surface of the separation membrane, a ratio of a peak strength of an infrared absorption peak around 1735 $cm^{-1}$, P1, to a peak strength of an infrared absorption peak at 1595 $cm^{-1}$, P2, P1/P2, is 0.015 or higher.
[4] The separation membrane for blood processing according to any of [1] to [3], wherein, in the general formula (1), $R^1$ is a hydrogen atom, $R^2$ is an ethyl group, n is 2, and m is 2.
[5] The separation membrane for blood processing according to any of [1] to [3], wherein, in the general formula (1), $R^1$ is a methyl group, $R^2$ is a methyl group, n is 2, and m is 2.
[6] The separation membrane for blood processing according to any of [1] to [3], wherein, in the general formula (1), $R^1$ is a methyl group, $R^2$ is an ethyl group, n is 2, and m is 2.
[7] The separation membrane for blood processing according to any of [1] to [3], wherein, in the general formula (1), $R^1$ is a hydrogen atom, $R^2$ is a methyl group, n is 3, and m is 1.
[8] The separation membrane for blood processing according to any of [1] to [3], wherein, in the general formula (1), $R^1$ is a hydrogen atom, $R^2$ is a methyl group, n is 4, and m is 1.
[9] The separation membrane for blood processing according to any of [1] to [3], wherein, in the general formula (1), $R^1$ is a hydrogen atom, $R^2$ is a methyl group, n is 5, and m is 1.
[10] The separation membrane for blood processing according to any of [1] to [3], wherein, in the general formula (1), $R^1$ is a hydrogen atom, $R^2$ is a methyl group, n is 6, and m is 1.
[11] A blood processing device comprising the separation membrane for blood processing according to any of [1] to [10].
[12] A method for producing a separation membrane for blood processing, the method comprising:

a step of forming a separation membrane containing polysulfone-based polymer and polyvinylpyrrolidone; and
a step of coating at least a part of the surface of the separation membrane with a coating solution containing a polymer material having a structure represented by the general formula (1).

[13] The method for producing a separation membrane for blood processing according to [12], wherein the coating solution contains water and an organic solvent, and the organic solvent is ethanol, methanol, or a mixture thereof.
[14] The method for producing a separation membrane for blood processing according to [12] or [13], wherein, in the step of forming the separation membrane,
the separation membrane is formed by using a membrane-forming dope containing polysulfone-based polymer and polyvinylpyrrolidone, and a ratio of polyvinylpyrrolidone to polysulfone-based polymer (polyvinylpyrrolidone/polysulfone-based polymer) in the membrane-forming dope is 27% by mass or less.
[15] A method for producing the blood processing device according to claim 11, the method comprising:

a step of forming a separation membrane containing polysulfone-based polymer and polyvinylpyrrolidone;

a step of potting to seal an inner space of the separation membrane from an outer space; and
a step of coating the surface of the separation membrane and the surface of the potting with a coating solution containing a polymer material having the structure represented by the general formula (1), wherein
the steps are performed in the order presented.

## Advantageous Effects of Invention

The separation membrane for blood processing and the blood processing device including the membrane of the present invention, can exert highly excellent blood compatibility even after being subjected to radiation sterilization in a dry state in the atmosphere.
Further, the separation membrane for blood processing of the present invention has good adhesion between the separation membrane and the blood-compatible polymer coated layer. For this reason, the separation membrane for blood processing of the present invention is expected to be free from problems such as degradation of the blood compatibility after a long-term use.
Furthermore, when blood from a living body in inflammatory conditions due to an infection or the like is processed, the separation membrane for blood processing of the present invention and the blood processing device including said membrane do not disrupt treatment because of reduced attachment of adhesive proteins to the separation membrane.
In addition, since a separation membrane can be thinly and uniformly coated with blood-compatible polymer in the separation membrane for blood processing of the present invention, a necessary and sufficient coating can be obtained with a small quantity of blood-compatible polymer.

## Brief Description of Drawings

[Figure 1] Figure 1 shows an infrared absorption curve obtained in attenuated total reflection-infrared spectroscopy (ATR-IR) for the surface of a separation membrane for blood processing of Example 1.
[Figure 2] Figure 2 shows a chromatogram obtained in pyrolysis gas chromatography-mass spectrometry for poly[2-(2-ethoxyethoxy)ethyl acrylate] (PEt2A).
[Figure 3] Figure 3 shows a chromatogram obtained in pyrolysis gas chromatography-mass spectrometry for a separation membrane for blood processing of Example 1.
[Figure 4] Figure 4 shows mass spectra of a separation membrane for blood processing of Example 1, with respect to peaks around chromatogram RT 7.9 (min). Based on an analysis of the spectra, it is identified as being the spectra of a chemical structure formula illustrated in the lower part (2-(2-ethoxyethoxy)ethyl alcohol).
[Figure 5] Figure 5 shows mass spectra of a separation membrane for blood processing of Example 9, with respect to peaks around chromatogram RT 12.7 (min). Based on an analysis of the spectra, it is identified as being the spectra of a chemical structure formula illustrated in the lower part (2-(2-methoxyethoxy)ethyl methacrylate).
[Figure 6] Figure 6 shows infrared absorption curves obtained in attenuated total reflection-infrared spectroscopy (ATR-IR) for the surface of a separation membrane for blood processing of Example 11 with a coating of poly[3-methoxypropyl acrylate].
[Figure 7] Figure 7 shows a chromatogram obtained in pyrolysis gas chromatography-mass spectrometry for poly[3-methoxypropyl acrylate].
[Figure 8] Figure 8 shows chromatograms obtained in pyrolysis gas chromatography-mass spectrometry for a separation membrane for blood processing of Example 11 with a coating of poly[3-methoxypropyl acrylate].
[Figure 9] Figure 9 shows mass spectra of poly[3-methoxypropyl acrylate] in Example 11, with respect to peaks around chromatogram RT 3.2 (min). Based on an analysis of the spectra, it is identified as being the spectra of a chemical structure formula illustrated in the lower part of trimethylene glycol monomethyl ether.
[Figure 10] Figure 10 is a photograph showing the surface condition of a hollow fiber separation membrane (PMC3A coating) after blood compatibility evaluation with inflammatory model blood in Example 22.
[Figure 11] Figure 11 is a photograph showing the surface condition of a hollow fiber separation membrane (PEt2A coating) after blood compatibility evaluation with inflammatory model blood in Example 22.
[Figure 12] Figure 12 is a photograph showing the surface condition of a hollow fiber separation membrane (no coating) after blood compatibility evaluation with inflammatory model blood in Comparative Example 5.

## Description of Embodiments

Hereinafter, modes for implementation of the present invention (hereinafter, referred to as "present embodiments") will be described in detail. The present invention is not limited to the following embodiments, and can be

implemented with various modifications without deviating from the gist.

The separation membrane for blood processing according to the present embodiments includes a separation membrane containing polysulfone-based polymer and polyvinylpyrrolidone, and a coated layer for imparting blood compatibility, the layer coated on at least a part of the surface of the separation membrane and containing a polymer material having the structure represented by the general formula (1) (hereinafter, occasionally referred to as "the polymer material of the general formula (1)", simply).

First, the separation membrane containing polysulfone-based polymer and polyvinylpyrrolidone will be described.

<Polysulfone-based polymer>

In the present embodiments, polysulfone-based polymer refers to polymer containing a sulfone ($-SO_2-$) group in the structure. Specific examples of polysulfone-based resin include polyphenylenesulfone, polysulfone, polyarylethersulfone, polyethersulfone, and copolymers thereof.

One polysulfone-based polymer may be used singly, or a mixture of two or more polysulfone-based polymers may be used.

Especially, polysulfone-based polymer represented by the following formula (a) or the following formula (b) is preferred from the viewpoint of control of fractionation properties.

$$(-Ar-SO_2-Ar-O-Ar-C(CH_3)_2-Ar-O-)n \qquad (a)$$

$$(-Ar-SO_2-Ar-O-)n \qquad (b)$$

In the formula (a) and the formula (b), Ar denotes a benzene ring; n indicates repetition of polymer, and is an integer of 1 or more.

Examples of polysulfone-based polymer represented by the formula (a) include commercially available products sold by Solvay S.A. under the name of "Udel (TM)" and that sold by BASF SE under the name of "Ultrason (TM)". Examples of polyethersulfone represented by the formula (b) include commercially available products sold by Sumitomo Chemical Co., Ltd. under the name of "SUMIKAEXCEL (TM)", for which there exist several types with different degrees of polymerization, etc., and they can be appropriately selected for use.

<Polyvinylpyrrolidone>

Polyvinylpyrrolidone is water-soluble hydrophilic polymer obtained by subjecting N-vinylpyrrolidone to vinyl polymerization, and widely used as a material for hollow fiber membranes as a hydrophilizing agent or a pore-forming agent.

Examples of polyvinylpyrrolidone include commercially available products sold by BASF SE under the name of "Luvitec (TM)", for which there exist several types with different molecular weights, and they can be appropriately used.

One polyvinylpyrrolidone may be used singly, or a mixture of two or more polyvinylpyrrolidones may be used.

In the present embodiments, the configuration in which the separation membrane contains polyvinylpyrrolidone is inferred to enhance the adhesion strength between the layer containing the polymer material represented by the general formula (1) and the separation membrane to thereby prevent the degradation of the blood compatibility after a long-term use.

The separation membrane may contain an additional component other than polysulfone-based polymer and polyvinylpyrrolidone. Examples of the additional component include polyhydroxyalkyl methacrylates such as polyhydroxyethyl methacrylate, polyhydroxypropyl methacrylate, and polyhydroxybutyl methacrylate, and polyethylene glycol. The content of the additional component is not limited, and may be 20% by mass or less, or may be 10% by mass or less, or may be 5% by mass or less. No additional component may be contained.

The ratio of polyvinylpyrrolidone to polysulfone-based polymer in the separation membrane in the present embodiments is preferably 42% by mass or less because the amount of elution of polyvinylpyrrolidone can be reduced, and the ratio is more preferably 27% by mass or less.

From the viewpoint of adhesion between the layer containing the polymer material represented by the general formula (1) and the separation membrane, on the other hand, the ratio of polyvinylpyrrolidone to polysulfone-based polymer is preferably 15% by mass or more, and more preferably 20% by mass or more. When the ratio of polyvinylpyrrolidone to polysulfone-based polymer is 18% by mass or more, the concentration of polyvinylpyrrolidone on the surface of the separation membrane can be controlled within a suitable range, which can enhance the effect to prevent protein adsorption, and thus can impart excellent blood compatibility to the separation membrane for blood processing.

Although the shape of the separation membrane is not limited, it is preferred for the separation membrane to

have a shape of a hollow fiber. From the viewpoint of permeation performance, it is more preferred for the separation membrane to be crimped.

Next, the layer containing the polymer material of the general formula (1) will be described.

The polymer material of the general formula (1) has polar groups of ether bonds and ester bonds that do not have strong electrostatic interactions with biological components, and does not have a large hydrophobic group in the molecular structure. By virtue of these features, the polymer material of the general formula (1) is a material which does not cause activation in blood even when being contacted with blood, what is called blood compatible material.

In particular, the polymer material of the general formula (1) is characterized by the side chain portion shown in the following general formula (1):

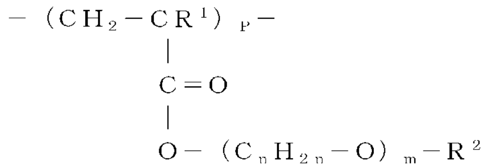
(1)

wherein $R^1$ is a hydrogen atom or a methyl group; $R^2$ is a methyl group or an ethyl group; n is 2 to 6 and m is 1 to 3; P denotes the number of repetition; and a plurality of each of $R^1$, $R^2$, n, and m present in one molecule may be the same or different.

The side chain having the above structure has high molecular mobility, and thus the polymer material having the side chain has low Tg and is expected to provide effects unique to the present invention.

Specifically, the side chain of the polymer material of the general formula (1) has high molecular mobility, and thus it is inferred that contact between the main chain and a biological component or the like contained in blood to be processed on the surface of the layer containing the polymer material is less likely to occur, and as a result the biocompatibility is enhanced and the adsorption and/or denaturation of adhesive proteins and platelets is insignificant.

The polymer material of the general formula (1) can have a plurality of side chains having different structures without deviating from the general formula (1). Further, the polymer material of the general formula (1) is only required to have the structure (repeating unit) represented by the general formula (1), and thus the polymer material of the general formula (1) may include, for example, a unit having a side chain structure of the general formula (1) where n = 1, without departing from the spirit of the present invention. However, it is preferable that the constitutional unit of the polymer material of the general formula (1) be at least acrylic acid, methacrylic acid, or a derivative thereof.

If one side chain having the structure shown in the general formula (1) is introduced per about 10 carbon atoms constituting the main chain, the polymer material of the general formula (1) can exert various features due to the side chain, and the features are more significantly exerted as the density of the side chains shown in the general formula (1) becomes higher. In particular, in the case that the main chain is an acrylic backbone (in the case that $R^1$ is a hydrogen atom), it follows that one side chain is introduced per two carbon atoms constituting the main chain, and thus the features due to the side chain can be significantly exerted.

Accordingly, one or more side chains shown in the general formula (1) are preferably included, more preferably two or more side chains shown in the general formula (1) are included, and even more preferably five or more side chains shown in the general formula (1) are included, per 10 carbon atoms constituting the main chain in the polymer material of the general formula (1).

The polymer material having the structure represented by the general formula (1) is polymer containing intermediate water, and not only the blood compatibility is good simply because ester bonds and ether bonds are present in the structure, but also the state of intermediate water adsorbed on the surface is expected to have a large impact on the blood compatibility. Further, the side chain shown in the general formula (1) has a high content of intermediate water in the case that n is 2 to 4, which allows the polymer material of the general formula (1) to have tendency to contain water therein to complicate adsorption of proteins or the like. In the case that n is 5 to 6, on the other hand, the polymer material of the general formula (1) exerts unique properties such as a property to adsorb proteins in an aqueous solution without causing denaturation while the biocompatibility is maintained.

In the case that the above m is 1, the specified characteristics are exerted in a wide range of temperature, and in the case that m is 2 or 3, the side chain becomes longer, which increases the variety of molecular motion, and may provide the polymer material with lower critical solution temperature (LCST) or upper critical solution temperature (UCST) etc., each a temperature at which the solubility in water drastically changes.

In the case that $R^1$ is hydrogen, the polymer material exhibits high hydrophilicity as a whole, and in the case that $R^1$ is a methyl group, the polymer material becomes hydrophobic, which is effective for imparting water-insoluble properties to the separation membrane.

While some of the polymer materials of the general formula (1) are known as biocompatible polymer, the present

inventors revealed that when a layer containing the polymer material of the general formula (1) is coated on the surface of a separation membrane containing polyvinylpyrrolidone, the separation membrane exhibits particularly excellent blood compatibility.

Further, the present inventors revealed that the polymer material of the general formula (1) exhibits good compatibility with blood from a living body in inflammatory conditions, as follows.

When inflammation is caused in a living body because of an infection or the like, vascular endothelial cells are activated, the amount of adhesive proteins in the blood increases in response to the damage, and the blood coagulation factor XII is activated, as a result of which the blood becomes easily coagulated. Accordingly, when such blood is contacted with a separation membrane, the adhesive proteins are attached to the surface of the separation membrane, and residual blood (a phenomenon that blood is coagulated and adheres to a separation membrane) frequently occurs. As a result, in dialysis, a trouble such as a lowered dialysis efficiency, disruption of dialysis treatment, and failure to return blood in a dialysis circuit is caused to bring a very serious situation.

However, even for blood in inflammatory conditions that causes such a serious situation, the polymer material of the general formula (1) exhibits good compatibility, and the above-mentioned troubles are less likely to occur presumably because the amount of adsorption of adhesive proteins is smaller for a separation membrane including this polymer material on the surface, or adhesive proteins are adsorbed thereon in a state such that they are easily released.

Further, the present inventors found that the blood compatibility of the separation membrane including the polymer material of the general formula (1) on the surface is dramatically enhanced when the abundance of the polymer material in the surface layer is high.

Since the separation membrane containing polysulfone-based polymer and polyvinylpyrrolidone is a porous body, the separation membrane may allow a coating solution applied onto the separation membrane to permeate the inside of the separation membrane through the pours. In particular, a larger pore size is employed in some cases depending on the type of a solvent for a coating solution, and, in this case, permeation is more likely to occur.

Depending on the type of a solvent for a coating solution, in some cases, a coating solution applied onto the separation membrane flows out of the surface and not much of the coating solution can remain on the surface.

Thus, the type and composition of a solvent for a coating solution containing the polymer material of the general formula (1) to be used in coating is considered to affect the amount of the polymer material of the general formula (1) being present in the surface layer of the separation membrane after application onto the separation membrane.

That is, the coating solution dissolving the polymer material of the general formula (1) therein is preferably the one that can remain on the surface of a porous separation membrane after being applied to allow the polymer material of the general formula (1) to remain on the surface.

The present inventors further studied, and found that in the case that the solvent for the coating solution is a mixture of water and an organic solvent, the amount of the polymer material of the general formula (1) to remain on the surface largely varies depending on the mixing ratio between water and the organic solvent.

Specifically, the polymer material of the general formula (1) is more likely to remain on the surface of the separation membrane as the mixing ratio of the organic solvent is smaller. The reason is not clear, however, it is presumed as follows: in the situation that the solvent for the coating solution can dissolve the polymer material of the general formula (1) therein, the polymer material of the general formula (1) is dissolved well in the coating solution when the mixing ratio of the organic solvent in the solvent is high, and thus the polymer material of the general formula (1) permeates the inside of the membrane on coating the separation membrane with the coating solution, and is less likely to remain on the surface; when the mixing ratio of the organic solvent is low, the solubility of the polymer material of the general formula (1) in the coating solution is low, and thus the polymer material of the general formula (1) precipitates from the coating solution and remains on the surface of the separation membrane when the coating solution are applied on the separation membrane and the balance of organic solvent/water in its solvent is disturbed , for example, by the organic solvent permeating the inside of the membrane in first.

In the case that the solvent for the coating solution is a mixture of water and an organic solvent, the mixing ratio of the organic solvent is preferably 80% by mass or less, more preferably 60% by mass or less, and even more preferably 40% by mass or less, provided that the polymer material of the general formula (1) is dissolved in the solvent, although the preferred ratio may change depending on the type of the polymer material of the general formula (1).

While it is known that, in ATR-IR, a wave entering into a sample penetrates through the sample to a slight depth and is reflected, and thus infrared absorption in a region within the depth of penetration can be measured, the present inventors found that the depth of the region to be measured in ATR-IR is almost equal to the depth of the above-mentioned "surface layer". That is, the present inventors conceived that the blood compatibility in a region within a depth almost equal to that of the region to be measured in ATR-IR represents the blood compatibility of the sample (separation membrane for blood processing), and a separation membrane for blood processing having a certain level of blood compatibility can be provided by including the polymer material of the general formula (1) in the region in a quantity equal to or more than a specific quantity (in other words, setting the quantity of the polymer material of the general formula (1) by using the peak strength derived from the polymer material of the general formula (1) in an infrared

absorption curve obtained in ATR-IR), and completed a more preferred mode of the present invention.

The region to be measured in ATR-IR depends on the wavelength of infrared light in the air, the incident angle, the refractive index of a prism, the refractive index of a sample, and so on, and is typically a region within 1 μm from the membrane surface.

The presence of the polymer material of the general formula (1) on the surface of the separation membrane can be confirmed through pyrolysis gas chromatography-mass spectrometry for the separation membrane. The presence of the polymer material of the general formula (1) is expected if a peak is found around 1735 $cm^{-1}$ in an infrared absorption curve obtained in attenuated total reflection-infrared spectroscopy (ATR-IR) for the surface of the separation membrane. However, the peak around 1735 $cm^{-1}$ may be derived from another substance.

Therefore, the presence of the polymer material of the general formula (1) on the surface can be established by performing pyrolysis gas chromatography-mass spectrometry to confirm a decomposition product of the polymer material of the general formula (1).

In order for the separation membrane for blood processing of the present embodiments to exhibit sufficient blood compatibility for practical use, the ratio of the peak strength of an infrared absorption peak corresponding to the ester group -O-C=O derived from the polymer material of the general formula (1) (around 1735 $cm^{-1}$), P1, to the peak strength of an infrared absorption peak corresponding to C=C (C=C in Ar) derived from polysulfone-based polymer (around 1595 $cm^{-1}$), P2, (P1/P2), each measured in ATR-IR, is preferably 0.015 or higher, more preferably 0.02 or higher, even more preferably 0.03 or higher, furthermore preferably 0.04 or higher, and particularly preferably 0.05 or higher.

Although the reason why the blood compatibility of the separation membrane for blood processing of the present embodiments is highly excellent is not clear, the occurrence of some interaction between polyvinylpyrrolidone (PVP) contained in the separation membrane and the polymer material of the general formula (1) (e.g., intermolecular tangling between PVP and the polymer material of the general formula (1)) is expected to be the cause.

In addition, PVP contained in the separation membrane has an effect to firmly fix the layer containing the polymer material of the general formula (1) onto the separation membrane. This effect is also expected to be due to the interaction described above.

The above-described peak strength ratio between the peak derived from the polymer material of the general formula (1) (around 1735 $cm^{-1}$) and the peak derived from polysulfone-based polymer (around 1595 $cm^{-1}$) (P1/P2) can be controlled through changing the composition of the solvent for the coating solution to be used in coating (specifically, the mixing ratio between the organic solvent and water). Specifically, the peak strength of the peak derived from the polymer material of the general formula (1) (around 1735 $cm^{-1}$) when ATR-IR is performed, P1, becomes weaker as the quantity of the organic solvent is larger, and the peak strength of the peak derived from the polymer material of the general formula (1) (around 1735 $cm^{-1}$), P1, becomes stronger as the quantity of the organic solvent is smaller.

The solubility of the polymer material of the general formula (1) in solvents is unique. In the case of poly[2-(2-ethoxyethoxy)ethyl acrylate] and poly[3-methoxypropyl acrylate], for example, they have different solubility in 100% ethanol, but both are soluble in a water/ethanol mixed solvent with a mixing ratio in a certain range. Within the mixing ratio in the range allowing dissolution, the peak strength of the peak corresponding to poly[2-(2-ethoxyethoxy)ethyl acrylate] or poly[3-methoxypropyl acrylate] (around 1735 $cm^{-1}$), P1, becomes stronger as the water content in the composition of the coating solution is larger.

In the case that the surface of the separation membrane containing polysulfone-based polymer and polyvinylpyrrolidone is coated with, for example, a layer containing poly[2-(2-ethoxyethoxy)ethyl acrylate] or poly[3-methoxypropyl acrylate], the state of the layer containing poly[2-(2-ethoxyethoxy)ethyl acrylate] or poly[3-methoxypropyl acrylate] present on the separation membrane can be evaluated by measuring UFR, one of indicators of water permeation performance.

When the separation membrane is coated with a layer containing poly[2-(2-ethoxyethoxy)ethyl acrylate] or poly[3-methoxypropyl acrylate], the porous membrane surface undergoes small variation in the pore size, and thus the water permeation performance is not greatly changed, which simplifies product design. This is presumably because the layer containing poly[2-(2-ethoxyethoxy)ethyl acrylate] or poly[3-methoxypropyl acrylate] attaches as an ultrathin film to the surface of the separation membrane to coat the separation membrane without greatly plugging the pores.

In the present embodiments, the number-average molecular weight of the polymer material of the general formula (1) is preferably 8,000 to 300,000. If the number-average molecular weight is 8,000 or lower, intermolecular tangling is partially insufficient, and the product tends to cause a larger amount of eluted substance. If the number-average molecular weight is 300,000 or higher, the handleability (stickiness and hardness) and solubility in solvents are poor, and insufficient dissolution tends to be observed. The number-average molecular weight is more preferably 10,000 to 250,000, and even more preferably 10,000 to 200,000.

In the present embodiments, the number-average molecular weight of the polymer material of the general formula (1) can be measured, for example, through gel permeation chromatography (GPC), as described in Examples.

To coat the layer containing the polymer material of the general formula (1) on the surface of the separation

membrane in the present embodiments, for example, a method in which the polymer material of the general formula (1) is mixed and dissolved in a membrane-forming (spinning) dope for use in formation of the separation membrane and then spinning is performed, a method in which the polymer material of the general formula (1) is mixed and dissolved in a bore liquid for use in formation of the separation membrane and then spinning is performed, or a method in which the separation membrane is coated with a coating solution dissolving the polymer material of the general formula (1) therein are suitably used.

Among these methods, the coating method in which the separation membrane is coated with a coating solution dissolving the polymer material of the general formula (1) therein is considered to be the most suitable, in view of the solubility of the polymer material of the general formula (1) in a membrane-forming dope and a bore liquid.

To coat the separation membrane with a coating solution dissolving the polymer material of the general formula (1) therein, the coating solution is allowed to flow through the separation membrane to come into contact with the surface thereof, suitably after the separation membrane is incorporated in a blood processing device and fixed.

The layer containing the polymer material of the general formula (1) is only required to be provided on at least a part of the surface of the separation membrane. Although it is preferable for the layer containing the polymer material of the general formula (1) to be provided on the whole surface of the separation membrane, it may be difficult to form the layer as a continuous layer. Accordingly, it is preferred to provide the layer containing the polymer material of the general formula (1) at least over the whole surface of the separation membrane.

The separation membrane for blood processing of the present embodiments can be subjected to sterilization treatment by using radiation sterilization, for example, even in an atmosphere with an oxygen concentration of 15% or more. Specifically, use of a deoxidant or purging with an inert gas such as nitrogen to lower the oxygen concentration is not required in subjecting the separation membrane for blood processing to radiation sterilization, and radiation sterilization can be performed in the atmosphere.

Next, the blood processing device will be described.

The blood processing device of the present embodiments is a blood processing device including the separation membrane for blood processing of the present embodiments, and can be used for blood purification therapy with extracorporeal circulation such as hemodialysis, hemofiltration, hemodiafiltration, blood fractionation, oxygenation, and plasmapheresis. In the blood processing device of the present embodiments, peeling off or the like of the layer containing the polymer material of the general formula (1) does not occur and the blood compatibility is very good even after the separation membrane for blood processing is subjected to radiation sterilization in the atmosphere, because firm bonds are formed between polyvinylpyrrolidone contained in the separation membrane and the polymer material of the general formula (1) by virtue of some interaction therebetween (e.g., an effect due to intermolecular tangling).

The blood processing device is preferably used, for example, as a hemodialyzer, hemofilter, or hemodiafilter, and more preferably used as any of them for continuous use, i.e., a continuous hemodialyzer, continuous hemofilter, or continuous hemodiafilter. The detail specification including the dimension and fractionation properties of the separation membrane is determined according to the application.

Next, a method for producing the blood processing device of the present embodiments will be described.

The method for producing the blood processing device of the present embodiments includes: a step of forming a separation membrane at least containing polysulfone-based polymer and polyvinylpyrrolidone; and a step of coating at least a part of the surface of the separation membrane with a coating solution containing the polymer material of the general formula (1), water, and an organic solvent.

The method can further include a step of drying the separation membrane to a moisture content of 10% by mass or less, or a step of performing radiation sterilization for the separation membrane for blood processing in an atmosphere with an oxygen concentration of 15% or more.

The separation membrane can be prepared through membrane formation by using a common method with a membrane-forming dope at least containing polysulfone-based polymer and polyvinylpyrrolidone.

The membrane-forming dope can be prepared by dissolving polysulfone-based polymer and polyvinylpyrrolidone in a solvent.

Examples of the solvent include dimethylacetamide, dimethylsulfoxide, N-methyl-2-pyrrolidone, dimethylformamide, sulfolane, and dioxane.

One solvent may be used singly, or a mixed solvent of two or more solvents may be used.

The concentration of polysulfone-based polymer in the membrane-forming dope may be any concentration, without any limitation, such that the concentration allows membrane formation and the membrane obtained has a performance as a permeable membrane. However, the concentration of polysulfone-based polymer in the membrane-forming dope is preferably 5 to 35% by mass, and more preferably 10 to 30% by mass. To achieve high water permeation performance, the concentration of polysulfone-based resin is preferably lower, and the concentration of polysulfone-based resin is more preferably 10 to 25% by mass.

The concentration of polyvinylpyrrolidone in the membrane-forming dope is not limited. However, the ratio of polyvinylpyrrolidone to polysulfone-based polymer (mass of polyvinylpyrrolidone / mass of polystyrene polymer) is adjusted preferably to 27% by mass or less, more preferably to 18 to 27% by mass, even more preferably to 20 to 27% by mass.

By adjusting the ratio of polyvinylpyrrolidone to polysulfone-based polymer to 27% by mass or less, the amount of elution of polyvinylpyrrolidone can be reduced. Suitably, by adjusting the ratio to 18% by mass or more, the concentration of polyvinylpyrrolidone on the surface of the separation membrane can be controlled in a suitable range, the effect to prevent protein adsorption can be enhanced, and an excellent blood compatibility can be imparted to the separation membrane for blood processing.

By using the membrane-forming dope as described above, a separation membrane as a sheet membrane or a hollow fiber membrane can be formed in accordance with a common method.

An example of methods for producing a hollow fiber membrane will be described.

A tube-in-orifice spinneret is used, and a membrane-forming spinning dope and a bore liquid to coagulate the membrane-forming spinning dope are simultaneously discharged to the air from the orifice and the tube of the spinneret, respectively. For the bore liquid, water or liquid mainly containing water can be used, and a mixed solution of a solvent used for a membrane-forming spinning dope and water is suitably used in general. For example, a 20 to 70% by mass aqueous solution of dimethylacetamide is used.

Each of the inner diameter and thickness of the hollow fiber membrane can be adjusted to a desired value through adjusting the discharge rate for the membrane-forming spinning dope and the discharge rate for the bore liquid.

The inner diameter of the hollow fiber membrane is not limited, and can be generally 170 to 250 $\mu$m and is preferably 180 to 220 $\mu$m for blood processing. From the viewpoint of the efficiency of diffusion and removal of low-molecular-weight substances through mass transfer resistance as a permeable membrane, the thickness of the hollow fiber membrane is preferably 50 $\mu$m or smaller.

From the viewpoint of strength, the thickness of the hollow fiber membrane is preferably 10 $\mu$m or larger.

The membrane-forming spinning dope discharged from the spinneret together with the bore liquid is allowed to run through an air gap portion, and then introduced into a coagulation bath provided below the spinneret and mainly containing water, and impregnated for a certain period, and thus the coagulation is completed. Then, the draft, which is represented by the ratio between the linear discharge rate of the membrane-forming spinning dope and the take-up speed, is preferably 1 or lower.

The air gap refers to a space between the spinneret and the coagulation bath, and the coagulation of the membrane-forming spinning dope is initiated from the inner surface side by the action of poor solvent components (poor solvent components to polysulfone-based polymer and polyvinylpyrrolidone) including water in the bore liquid simultaneously discharged from the spinneret. To form a separation membrane with a smooth surface and stabilize the structure of the separation membrane on the initiation of coagulation, the draft is preferably 1 or lower, and more preferably 0.95 or lower.

The solvent remaining in the hollow fiber membrane is then removed through washing with hot water or the like, and thereafter the hollow fiber membrane is continuously introduced into a dryer and dried with hot air or the like, and thus a dried hollow fiber membrane can be obtained. The washing is intended for removal of extra polyvinylpyrrolidone, and is preferably performed with hot water of 60°C or higher for 120 seconds or longer, and is more preferably performed with hot water of 70°C or higher for 150 seconds or longer.

To embed with urethane resin in a later step, and, in the case of the present embodiments, to perform radiation sterilization in a dry state, it is preferred to control the moisture content of the separation membrane to 10% by mass or less by drying.

The hollow fiber membrane obtained through the above steps can be subjected to a step of module production as a bundle with the length and the number of the hollow fibers adjusted so as to achieve a desired membrane area. In this step, the hollow fiber membrane is packed in a cylindrical container having two nozzles near each end of the side surface, and each end is embedded with urethane resin.

Subsequently, each end portion of the cured urethane is cut off to make each end into an end with openings from the hollow fiber membranes (end with the hollow fiber membranes being exposed). To each of the ends, header cap with a nozzle for introduction (discharge) of liquid is attached, and the resultant is set up into a shape of a blood processing device.

After a module is set up as described above, a layer containing polyvinylpyrrolidone can be formed on the surface of the separation membrane through injection of a coating solution containing the polymer material of the general formula (1) to the inside of the hollow fiber membrane.

Next, the method for forming the layer containing the polymer material of the general formula (1) on the surface of the separation membrane will be described.

In the present embodiments, for example, the layer can be formed by applying a coating solution containing the polymer material of the general formula (1) onto the surface of the separation membrane.

The coating solution may be any solvent which does not dissolve polysulfone-based polymer therein and dissolves or disperses the polymer material of the general formula (1) therein, without any limitation. Since the polymer

material of the general formula (1) has strong affinity to the separation membrane by virtue of, for example, interaction with polyvinylpyrrolidone contained in the separation membrane, the layer can be easily formed regardless of the type of the coating solution. However, water or an aqueous solution of alcohol is preferred from the viewpoint of safety in the step and handleability in the subsequent step of drying. From the viewpoint of the boiling point and toxicity, water, an aqueous solution of ethanol, an aqueous solution of methanol, an aqueous solution of isopropyl alcohol, and so on, are suitably used.

The type and composition of the solvent for the coating solution need to be considered in association with the separation membrane as a substrate to be coated, as described above, in order to increase the abundance of the polymer material of the general formula (1) in the surface layer.

The concentration of the polymer material of the general formula (1) in the coating solution is not limited, and can be, for example, 0.001 % by mass to 1% by mass, and is preferably 0.005% by mass to 0.3% by mass, with respect to the coating solution.

The method for applying the coating solution is not limited, and, for example, a method can be employed in which the coating solution is injected from a header cap with a nozzle onto the separation membrane and excessive solution is then removed by using compressed air.

It is preferred to perform drying after application, and the method for drying is not limited, and drying may be performed under reduced pressure or under heating until a constant weight is achieved. The temperature in drying under heating is only required to be a temperature such that members of a module are not degraded, where the temperature can be appropriately set only with consideration of the balance between the temperature and the duration of the step.

The thus-obtained separation membrane for blood processing containing the polymer material of the general formula (1) on the surface of the separation membrane can be subjected to radiation sterilization treatment. The atmosphere for radiation sterilization treatment is not limited, and radiation sterilization can be performed in an atmosphere with an oxygen concentration of 15% or more, or even in the atmosphere, without causing denaturation or the like of the separation membrane.

To perform radiation sterilization, electron beams, $\gamma$-rays, X-rays, and so on can be used, and any of them may be used. In the case of an electron beam or a $\gamma$-ray, the exposure dose of radiation is typically 15 to 50 Kgy, and irradiation is performed preferably in a dose range of 15 to 40 Kgy or 20 to 40 Kgy. After a step of sterilization such as radiation sterilization in this manner, a blood processing device is completed.

## Examples

Hereinafter, the present invention will be specifically described with reference to Examples and Comparative Examples. However, the present invention is never limited to these Examples.

### (1) Infrared ATR (attenuated total reflection) measurement

The procedure of sampling was as follows.

The inner surface of a hollow fiber-shaped separation membrane was washed with distilled water at 100 mL/min per 1.5 $m^2$ for 5 minutes for priming. The blood processing device after priming was taken apart to take out the hollow fiber for a sample, which was cut out with a razor and the surface of the hollow fiber separation membrane was turned upward, and 10 points were arbitrarily selected therefrom and a prism was pressed onto each point to measure the infrared ATR. (650 $cm^{-1}$ to 4000 $cm^{-1}$) The prism used was an ATR-30-Z (ZnSe, refractive index: 2.4) manufactured by JASCO Corporation, and the incident angle was 60°.

The area of an infrared absorption peak corresponding to the ester group -O-C=O derived from the polymer material of the general formula (1) around 1735 $cm^{-1}$ (peak area when setting a line connecting 1715 $cm^{-1}$ and 1755 $cm^{-1}$ as a base line) was defined as P1, and the area of an infrared absorption peak corresponding to C=C derived from polysulfone-based around 1595 $cm^{-1}$ (peak area when setting a line connecting 1555 $cm^{-1}$ and 1620 $cm^{-1}$ as a base line) was defined as P2, and the abundance of the polymer material of the general formula (1) on the surface of the separation membrane was determined from the average value of the ratio P1/P2.

### (2) Pyrolysis gas chromatography-mass spectrometry

Pyrolysis gas chromatography-mass spectrometry was performed by using the following apparatuses and conditions.

Name of apparatus: Agilent 5973N-MSD (manufactured by Agilent Technologies, Inc.)
Name of pyrolyzer: Double-shot pyrolyzer Py-2020iD (manufactured by Frontier Laboratories Ltd.)
Name of column: HP-5MS

Specification of column: length of 30 m, inner diameter of 0.25 mm, membrane thickness of 0.25 μm, membrane of phenylmethylsiloxane Pyrolysis temperature/duration: 600°C/0 sec
Interface temperature of pyrolyzer: 320°C
Injection temperature for GC: 320°C
Oven initial temperature/retention time for GC: 40°C/3 min
Oven temperature elevation rate for GC: 10°C/min
Oven end-point temperature/retention time: 300°C/0 min
Transfer line temperature for MS: 300°C
Ionization source temperature for MS: 230°C
Quadrupole temperature for MS: 150°C
Ionization voltage/current for MS: 70 eV/35 μA
Scanning range for MS: 29 - 550

(3) Measurement of UFR (mL/Hr·mmHg)

An original solution (Urea = 1000 ppm, VB-12 (vitamin B12) = 10 ppm/pure water) was allowed to flow through the blood processing device from the inlet for blood (bin) to the outlet for blood (bout) at 100 mL/min, and pure water was allowed to flow the blood processing device from the inlet for dialysate (din) to the outlet for dialysate (dout) at 500 mL/min in the direction opposing to the flow of the original solution.

UFR is represented by the following equation.

$$\text{UFR (mL/Hr·mmHg)} = \{\text{flow rate at bin (mL/min)} \times 60\ \text{(min/Hr)} \times \text{UFR coefficient}\} / \text{TMP} = \{100 \times 60 \times \text{UFR coefficient}\} / \text{TMP}$$

Here, the UFR coefficient is a reference pressure for calculation of a UFR measurement. TMP (mmHg) is a pressure applied to the separation membrane for blood processing when the outlet for blood (bout) is blocked, and represented by the following equation.

$$\text{TMP} = \{(\text{Pbin} + \text{Pbout}) - (\text{Pdin} + \text{Pdout})\} / 2$$

(4) Measurement of contact angle

The inner surface of a hollow fiber-shaped separation membrane was washed with distilled water at 100 mL/min per 1.5 $m^2$ for 5 minutes for priming. The blood processing device after priming was taken apart to take out the hollow fiber for a sample, which was cut out with a razor and the surface of the hollow fiber separation membrane was turned upward, and the contact angle was measured.

Then, priming as washing at 100 mL/min per 1.5 $m^2$ for 5 minutes was repeated five times, and the surface of the hollow fiber separation membrane was checked for the presence or absence of change in the contact angle.

(5) Blood compatibility - measurement of lactate dehydrogenase (LDH) activity and number of hollow fibers with residual blood

The blood compatibility of the separation membrane was evaluated on the basis of attachment of platelets to the membrane surface, and quantified by using the activity of lactate dehydrogenase (LDH) contained in platelets attached to the membrane as an indicator.

The blood processing device was washed with saline (OTSUKA NORMAL SALINE, Otsuka Pharmaceutical Co., Ltd.) for priming. The blood processing device after priming was taken apart to take out the separation membrane, and both ends of the separation membrane were processed with epoxy resin (Bond Quick Set, Konishi Co., Ltd.) so that the effective length was 15 cm and the area of the inner surface of the membranes was $5 \times 10^{-3}$ $m^2$ to produce a mini-module. For washing, 10 mL of saline was allowed to flow through the inside of the hollow fibers of the mini-module.

Thereafter, 15 mL of human blood with heparin (heparin: 1000 IU/L) was circulated in the thus-produced mini-module at a flow rate of 1.3 mL/min at 37°C for 4 hours. The inside of the mini-module was washed with 10 mL of saline, and the outside of the mini-module was washed with 10 mL of saline. Half of the whole hollow fiber membranes with a length of 7 cm was taken out of the washed mini-module, and then shredded, and the resultant was put in a Spitz tube

for LDH measurement, which was used as a measurement sample. The number of hollow fibers with generation of residual blood (coagulates of blood in a hollow fiber) in the mini-module was visually determined.

Subsequently, 0.5 mL of 0.5 vol% TritonX-100/PBS solution obtained by dissolving TritonX-100 (NACALAI TESQUE, INC.) in phosphate buffer solution (PBS) (Wako Pure Chemical Industries, Ltd.) was added to the Spitz tube for LDH measurement, and shaking was performed for 60 minutes to crush cells (mostly, platelets) attached to the separation membrane, and LDH in the cells was extracted. The extract was aliquoted into 0.05 mL portion, and 2.7 mL of 0.6 mM sodium pyruvate solution and 0.3 mL of 1.277 mg/mL nicotinamide adenine dinucleotide (NADH) solution were added to the aliquot and reacted at 37°C for 1 hour, and thereafter the absorbance at 340 nm was measured.

Absorbance was measured in the same manner for a separation membrane which had not been reacted with blood (blank), and the difference in absorbance, ΔAbs (340 nm)/Hr, was calculated by using the following equation.

ΔAbs (340 nm)/Hr

= [Abs (340 nm) of blank (membrane without contact with blood) after 1 Hr] - [Abs (340 nm) of sample (membrane with contact with blood) after 1 Hr]

Then, ΔAbs (340 nm)/Hr was measured in the same manner for a separation membrane containing polysulfone-based polymer and polyvinylpyrrolidone alone (herein, Comparative Example 1) as a control sample, and the value was assumed as 100 and a proportional value was calculated.

In the present method, the proportional value was used as the LDH activity of each sample. High LDH activity indicates a large amount of attachment of platelets to the membrane surface and low blood compatibility. The measurement was performed three times, and the average value was recorded.

As a separation membrane excellent in blood compatibility, those with an LDH activity of lower than 25 are preferred, and those with an LDH activity of 5 or lower are more preferred, and those with an LDH activity of 2 or lower can be deemed to be highly excellent.

(6) Evaluation test with inflammatory model blood

Patients requiring treatment with a hemodialyzer, a hemofilter, a hemodiafilter, or the like are typically more or less in inflammatory conditions. It has been known that, in the blood of a patient in inflammatory conditions, production of inflammatory markers and the coagulation system are accelerated through activation, and the properties are largely different from the properties of the blood of a healthy individual, and it has been revealed that when the blood of a patient in inflammatory conditions is processed with a separation membrane, useful proteins including albumin and fibrinogen are likely to be adsorbed on the separation membrane.

Accordingly, it is useful in evaluation of blood compatibility to perform the following evaluation with inflammatory model blood, in addition to evaluation with blood from a healthy individual.

(6-1) Method for preparing inflammatory model blood

Inflammation model blood was prepared by using a method disclosed by Yasuda et al. (N. Yasuda, et al., J. Surg. Research, 176, 2012).

Specifically, blood was collected from a healthy individual with 1000 IU/L of heparin as an anticoagulant, and LPS: Lipopolysaccharide (derived from 0-127, Sigma-Aldrich Co., LLC.) was then added to the blood to a blood level of $1.0 \times 10^{-4}$ mg/mL, and thereafter the resultant was incubated at 39°C for 1.5 hours, and thus inflammatory model blood was prepared.

(6-2) Evaluation and indicator of blood compatibility with inflammatory model blood

As a blood pool, 20 mL of the inflammatory model blood prepared above was used and it was allowed to pass through a hollow fiber-type blood processing device with the area of the inner surface of the membranes adjusted to $5 \times 10^{-3}$ $m^2$ at a flow rate of 1.0 mL/min for 30 minutes, and the blood was then returned with 10 mL of saline. After the blood return, the hollow fiber membrane-type blood processing device was taken apart to take out the separation membrane, and a part of the separation membrane corresponding to a membrane area of 15 $cm^2$ was shredded, and the resultant was put in a microtube containing 2 mL of 1% SDS solution (sodium laurylsulfate solution), and subjected to

extraction with shaking at 1850 rpm for 1 hour, which was used as a sample for measuring the amount of attachment of proteins on the separation membrane.

The protein concentration of the extract was measured by using a BCA Protein Assay Kit (manufactured by Thermo Fisher Scientific Inc. (Waltham, MA, USA)), and the total amount of attachment of proteins per 1 mL of the extract was calculated.

(7) Preparation of polymer material having structure represented by general formula (1)

(A) Preparation of PEt2A (poly[2-(2-ethoxyethoxy)ethyl acrylate])

In 60 g of 1,4-dioxane, 15 g of 2-(2-ethoxyethoxy)ethyl acrylate was polymerized by using azobisisobutyronitrile (0.1% by weight) as an initiator with nitrogen bubbling at 75°C for 10 hours. After the completion of polymerization reaction, the polymerization solution obtained was added dropwise to n-hexane to precipitate and isolate the product. The product obtained was dissolved in tetrahydrofuran, and purification with n-hexane was further performed twice. The purified product was dried under reduced pressure for a whole day to obtain colorless, transparent, syrupy polymer. The yield amount (percentage yield) was 12.0 g (80.0%).

The structure of the polymer obtained was determined by using $^1$H-NMR.

From results of molecular weight analysis with GPC, the number-average molecular weight (Mn) was found to be 11,600, and the molecular weight distribution (Mw/Mn) was found to be 3.9.

(B) Preparation of PMe2MA (poly[2-(2-methoxyethoxy)ethyl methacrylate])

In 50 g of 1,4-dioxane, 10 g of 2-(2-methoxyethoxy)ethyl methacrylate was polymerized by using azobisisobutyronitrile (0.1% by weight) as an initiator with nitrogen bubbling at 80°C for 8 hours. After the completion of polymerization reaction, the polymerization solution obtained was added dropwise to n-hexane to precipitate and isolate the product. The product obtained was dissolved in tetrahydrofuran, and purification with n-hexane was further performed twice. The purified product was dried under reduced pressure for a whole day to obtain colorless, transparent, syrupy polymer. The yield (percentage yield) was 8.2 g (82.0%).

The structure of the polymer obtained was determined by using $^1$H-NMR.

From results of molecular weight analysis with GPC, the number-average molecular weight (Mn) was found to be 104,300, and the molecular weight distribution (Mw/Mn) was found to be 4.6.

(C) Preparation of PEt2MA (poly[2-(2-ethoxyethoxy)ethyl methacrylate])

In 60 g of 1,4-dioxane, 15 g of 2-(2-ethoxyethoxy)ethyl methacrylate was polymerized by using azobisisobutyronitrile (0.1% by weight) as an initiator with nitrogen bubbling at 75°C for 2 hours. After the completion of polymerization reaction, the polymerization solution obtained was added dropwise to n-hexane to precipitate and isolate the product. The product obtained was dissolved in tetrahydrofuran, and purification with n-hexane was further performed twice. The purified product was dried under reduced pressure for a whole day to obtain colorless, transparent, syrupy polymer. The yield (percentage yield) was 5.2 g (34.7%).

The structure of the polymer obtained was determined by using $^1$H-NMR.

From results of molecular weight analysis with GPC, the number-average molecular weight (Mn) was found to be 142,500, and the molecular weight distribution (Mw/Mn) was found to be 6.1.

(D) Preparation of PMC3A (poly[3-methoxypropyl acrylate])

(D-1) Synthesis of 3-methoxypropyl acrylate (see the following formula)

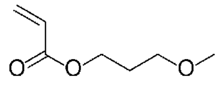

Under nitrogen gas flow, 15.5 g (153 mmol) of triethylamine, 13.5 g (150 mmol) of 3-methoxy-1-propanol, and 200 mL of diethyl ether were added into a three-necked eggplant flask (capacity: 500 mL), and the reaction system was cooled to 0°C in an ice water bath. To the reaction system, 14.0 g (155 mmol) of acryloyl chloride was added dropwise with stirring over 30 minutes, and then the reaction system was stirred at room temperature for 12 hours. After the

completion of the reaction was confirmed by using $^1$H NMR, the reaction was quenched. White precipitates formed with the progression of the reaction were removed through suction filtration, and the reaction solvent was distilled off from the resulting filtrate with a rotary evaporator to obtain the reaction product as a liquid. The reaction product obtained was separated and purified by using silica gel column chromatography (eluent: hexane:diethyl ether = 100:0 to 90:10), and then purified through distillation under reduced pressure in the presence of calcium hydride to obtain 9.95 g (69.1 mmol, percentage yield: 46% (in terms of MC3A)) of a transparent liquid.

The boiling point was 24.5°C to 25.5°C/0.08 mmHg, and $^1$H-NMR (500 MHz, $CDCl_3$) analysis identified the transparent liquid as 3-methoxypropyl acrylate.

The result of the $^1$H-NMR analysis is shown in the following. $^1$H-NMR (500 MHz, $CDCl_3$): $\delta$ = 6.39 (d, J = 17.3 Hz, 1H), 6.11 (dd, J = 17.3 Hz, 10.4 Hz, 1H), 5.81 (d, J = 10.4 Hz, 1H), 4.24 (t, J = 6.4 Hz, 2H), 3.45 (t, J = 6.3 Hz, 2H), 3.33 (s, 3H), 1.93 (p, J = 6.4 Hz, 2H). $^{13}$C-NMR (125 MHz, $CDCl_3$): $\delta$ = 166.2, 130.6, 128.5, 69.1, 61.7, 58.7, 29.0.

(D-2) Production of poly[3-methoxypropyl acrylate] (see the following formula)

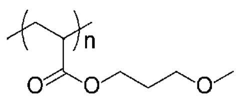

Into a three-necked eggplant flask (capacity: 100 mL), 7.50 g (52.0 mmol) of 3-methoxypropyl acrylate obtained above, 30.2 g of 1,4-dioxane, and 7.5 mg (0.047 mmol) of azobisisobutyronitrile were added. The reaction solution was stirred for 30 minutes while dry nitrogen gas was allowed to pass through the reaction solution, and thus the reaction system was purged with nitrogen. The bottom of the three-necked eggplant flask was soaked in an oil bath with the temperature set at 75°C, and polymerization was performed through stirring under nitrogen gas flow for 6 hours. After the progression of the polymerization reaction was determined by using $^1$H NMR and a sufficiently high conversion rate (around 90%) was confirmed, the polymerization system was allowed to cool to room temperature to quench the reaction. The reaction solution was added dropwise to hexane to precipitate the polymer, and the supernatant was removed through decantation, and the precipitate was dissolved in tetrahydrofuran for recovery. After dissolving in tetrahydrofuran, an operation of reprecipitation with hexane was performed twice for purification, and the resulting precipitate was further stirred in water for 24 hours. The water was removed through decantation, and the precipitate was dissolved in tetrahydrofuran for recovery. The solvent was distilled off under reduced pressure, and the resultant was dried with a vacuum dryer to obtain 6.47 g of polymer (percentage yield: 86% (in terms of PMC3A)).

The molecular weight was measured using a part of the polymer obtained, and the number-average molecular weight (Mn) was found to be 31000 and the molecular weight distribution (Mw/Mn) was found to be 2.5. The glass transition temperature of the polymer was measured to be -48.0°C, and $^1$H-NMR (500 MHz, $CDCl_3$) analysis identified the polymer as poly[3-methoxypropyl acrylate].

The result of the $^1$H-NMR analysis is shown in the following. $^1$H-NMR (500 MHz, $CDCl_3$): $\delta$ = 4.10 (br, 2H), 3.41 (brt, 2H), 3.31 (s, 3H), 2.26 (s, 1H), 1.86-1.62 (m, 4H).$^{13}$C-NMR (125 MHz, $CDCl_3$): $\delta$ = 174.3, 69.1, 62.0, 58.6, 41.5, 29.0, 0.07.

(E) Preparation of PMC4A (poly[4-methoxybutyl acrylate])

(E-1) Synthesis of 4-methoxybutyl acrylate (see the following formula)

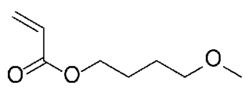

(E-1-1) Synthesis of 4-methoxy-1-butanol

Under nitrogen gas flow, 53.6 g (600 mmol) of 1,4-butanediol and 300 mL of tetrahydrofuran were added into a three-necked eggplant flask (capacity: 500 mL), and 18.1 g (450 mmol) of sodium hydride was added thereto in small portions while stirring was performed with cooling, as necessary. After the completion of addition of the total quantity of sodium hydride, the resultant was stirred at room temperature for 1 hour, and 63.4 g (450 mmol) of iodomethane was

added dropwise thereto, and the resultant was further stirred for 14 hours. After the progression of the reaction was confirmed by using $^1H$ NMR, a small quantity of water was added thereto to quench the reaction. The solution was acidified with 2 N hydrochloric acid, and tetrahydrofuran was then distilled off with a rotary evaporator. Diethyl ether was added to the resulting reaction mixture for dilution, and anhydrous magnesium sulfate was then added thereto to dry the reaction mixture. From the diethyl ether solution dried, magnesium sulfate and precipitates were removed through suction filtration, and the resulting filtrate was concentrated with a rotary evaporator. The resulting concentrate was separated and purified by using silica gel column chromatography (hexane, dichloromethane, and methanol were sequentially used as eluent), and then concentrated to obtain 18.5 g (178 mmol, percentage yield: 29.6%) of a transparent liquid. The boiling point was 50.0°C/0.08 mmHg, and $^1H$-NMR (500 MHz, $CDCl_3$) analysis identified the transparent liquid as poly[4-methoxy-1-butanol].

The result of the $^1H$-NMR analysis is shown in the following. $^1H$-NMR (500 MHz, $CDCl_3$): $\delta$ = 3.58 (t, J = 6.0 Hz, 2H), 3.38 (t, J = 6.0 Hz, 2H), 3.31 (s, 3H), 2.20 (s, 1H), 1.61 (m, 4H).$^{13}C$-NMR (125 MHz, $CDCl_3$): $\delta$ = 72.8, 62.6, 58.6, 30.1, 26.7.

(E-1-2) Synthesis of 4-methoxybutyl acrylate

Synthesis was performed in the same manner as in (D-1) except that 15.8 g (150 mmol) of 4-methoxy-1-butanol synthesized in (E-1-1) was used and the quantities of triethylamine, diethyl ether, and acryloyl chloride were changed to 17.5 g (165 mol), 250 mL, and 14.5 g (158 mmol), respectively, and 11.4 g (72.2 mmol, percentage yield: 48%) of a transparent liquid was obtained.

The boiling point was 50.0°C/0.08 mmHg, and $^1H$-NMR (500 MHz, $CDCl_3$) analysis identified the transparent liquid as 4-methoxybutyl acrylate.

The result of the $^1H$-NMR analysis is shown in the following. $^1H$-NMR (500 MHz, $CDCl_3$): $\delta$ = 6.47 (d, J = 15 Hz, 1H), 6.20 (dd, J = 8.75 Hz, 5.0 Hz, 1H), 5.89 (d, J = 10.5 Hz, 1H), 4.26 (t, J = 6.5 Hz, 2H), 3.49 (t, J = 6.5 Hz, 2H), 3.42 (s, 3H), 1.84-1.75 (m, 4H).$^{13}C$-NMR (125 MHz, $CDCl_3$): $\delta$ = 166.3, 130.5, 128.6, 72.2, 64.6, 58.6, 26.2, 25.5.

(E-2) Production of poly[4-methoxybutyl acrylate] (see the following formula)

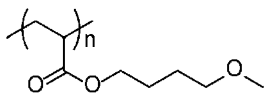

Synthesis was performed in the same manner as in (D-2) except that 9.41 g (59.5 mmol) of 4-methoxybutyl acrylate obtained above, 41.2 g of 1,4-dioxane, and 10 mg (0.061 mmol) of azobisisobutyronitrile were used and the polymerization time was changed to 8 hours, and 7.21 g (percentage yield: 77% (in terms of PMC4A)) of polymer was obtained.

The molecular weight was measured using a part of the thus-obtained polymer by using a method described later, and the number-average molecular weight (Mn) was found to be 29000 and the molecular weight distribution (Mw/Mn) was found to be 2.2. The glass transition temperature of the polymer was measured to be -64.6°C, and $^1H$-NMR (500 MHz, $CDCl_3$) analysis identified the polymer as poly[4-methoxybutyl acrylate].

The result of the $^1H$-NMR analysis is shown in the following. $^1H$-NMR (500 MHz, $CDCl_3$): $\delta$ = 4.03 (br, 2H), 3.37 (t, J = 6.0 Hz, 2H), 3.30 (s, 3H), 2.24 (s, 1H), 1.87-1.59 (m, 6H). $^{13}C$-NMR (125 MHz, $CDCl_3$): $\delta$ = 174.3, 72.1, 64.4, 58.5, 41.4, 35.0, 26.1, 25.4.

(F) Preparation of PMC5A (poly[5-methoxypentyl acrylate])

(F-1) Synthesis of 5-methoxypentyl acrylate (see the following formula)

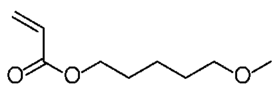

(F-1-1) Synthesis of 5-methoxy-1-pentanol

Synthesis was performed in the same manner as in (E-1-1) except that 31.4 g (300 mmol) of 1,5-pentanediol was used and the quantities of tetrahydrofuran, sodium hydride, and iodomethane were changed to 200 mL, 12.3 g (300 mmol), and 43.8 g (300 mmol), respectively, and 14.4 g (122 mmol, percentage yield: 41%) of a transparent liquid was obtained. The boiling point was 60°C to 64°C/0.08 mmHg, and $^1$H-NMR (500 MHz, $CDCl_3$) analysis identified the transparent liquid as 5-methoxy-1-pentanol.

The result of the $^1$H-NMR analysis is shown in the following. $^1$H-NMR (500 MHz, $CDCl_3$): $\delta$ = 3.61 (m, 2H), 3.36 (t, J = 7.5 Hz, 2H), 3.31 (s, 3H), 1.85-1.35 (m, 7H). $^{13}$C-NMR (125 MHz, $CDCl_3$): $\delta$ = 72.8, 62.6, 58.6, 32.5, 29.3, 22.4.

(F-1-2) Synthesis of 5-methoxypentyl acrylate

Synthesis was performed in the same manner as in (D-1) except that 15.4 g (130 mmol) of 5-methoxy-1-pentanol synthesized in (F-1-1) was used and the quantities of triethylamine, diethyl ether, and acryloyl chloride were changed to 14.5 g (143 mol), 200 mL, and 12.4 g (136.5 mmol), respectively, and 5.95 g (34.6 mmol, percentage yield: 27%) of a transparent liquid was obtained. The boiling point was 58°C to 71°C /0.08 mmHg, and $^1$H-NMR (500 MHz, $CDCl_3$) analysis identified the transparent liquid as 5-methoxy-1-pentanol.

The result of the $^1$H-NMR analysis is shown in the following. $^1$H-NMR (500 MHz, $CDCl_3$): $\delta$ = 6.36 (d, J = 18.0 Hz, 1H), 6.11 (dd, J = 5.0 Hz, 8.8 Hz, 1H), 5.79 (d, J = 9.5 Hz, 1H), 4.17 (t, J = 7.0 Hz, 2H), 3.38 (t, J = 6.3 Hz, 2H), 3.31 (s, 3H), 1.67-1.58 (m, 4H), 1.43 (m, 2H). $^{13}$C-NMR (125 MHz, $CDCl_3$): $\delta$ = 166.4, 130.6, 128.6, 72.6, 64.6, 58.6, 29.3, 28.5, 22.7.

(F-2) Production of poly[5-methoxypentyl acrylate] (see the following formula)

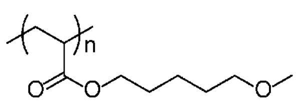

Synthesis was performed in the same manner as in (D-2) except that 5.01 g (32.1 mmol) of 5-methoxypentyl acrylate obtained above, 20 g of 1,4-dioxane, and 5 mg (0.030 mmol) of azobisisobutyronitrile were used and the polymerization time was changed to 8 hours, and 3.64 g (percentage yield: 73%) of polymer was obtained.

The molecular weight was measured using a part of the thus-obtained polymer by using a method described later, and the number-average molecular weight (Mn) was found to be 50000 and the molecular weight distribution (Mw/Mn) was found to be 2.3. The glass transition temperature of the polymer was measured to be -77.6°C, and $^1$H-NMR (500 MHz, $CDCl_3$) analysis identified the polymer as poly[5-methoxy-1-pentanol].

The result of the $^1$H-NMR analysis is shown in the following. $^1$H-NMR (500 MHz, $CDCl_3$): $\delta$ = 4.00 (br, 2H), 3.36 (t, J = 6.5 Hz, 2H), 3.31 (s, 3H), 2.24 (m, 1H), 1.87-1.38 (m, 8H). $^{13}$C-NMR (125 MHz, $CDCl_3$): $\delta$ = 174.3, 72.5, 64.6, 58.6, 41.5, 29.3, 28.5, 23.5.

(G) Preparation of PMC6A (poly[6-methoxyhexyl acrylate])

(G-1) Synthesis of 6-methoxyhexyl acrylate (see the following formula)

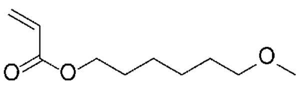

(G-1-1) Synthesis of 6-methoxy-1-hexanol

Synthesis was performed in the same manner as in (E-1-1) except that 35.6 g (300 mmol) of 1,6-hexanediol was used and the quantities of tetrahydrofuran, sodium hydride, and iodomethane were changed to 230 mL, 12.4 g (300 mmol), and 42.6 g (300 mmol), respectively, and 10.2 g (77.2 mmol, percentage yield: 26%) of a transparent liquid was obtained. The boiling point was 94.0°C to 100°C /0.08 mmHg, and $^1$H-NMR (500 MHz, $CDCl_3$) analysis identified the

transparent liquid as 6-methoxy-1-hexanol.

The result of the $^1H$-NMR analysis is shown in the following. $^1H$-NMR (500 MHz, $CDCl_3$): δ = 3.62 (t, J = 6.5 Hz, 2H), 3.35 (t, J = 6.8 Hz, 2H), 3.31 (s, 3H), 1.65-1.36 (m, 9H). $^{13}C$-NMR (125 MHz, $CDCl_3$): δ = 72.8, 62.8, 58.5, 32.7, 29.6, 25.9, 25.6.

(G-1-2) Synthesis of 6-methoxyhexyl acrylate

Synthesis was performed in the same manner as in (D-1) except that 9.25 g (70.0 mmol) of 6-methoxy-1-hexanol synthesized in (G-1-1) was used and the quantities of triethylamine, diethyl ether, and acryloyl chloride were changed to 6.65 g (73.5 mol), 200 mL, and 6.65 g (73.5 mmol), respectively, and 5.77 g (31.0 mmol, percentage yield: 44%) of a transparent liquid was obtained. The boiling point was 99°C to 103°C/0.08 mmHg, and $^1H$-NMR (500 MHz, $CDCl_3$) analysis identified the transparent liquid as 6-methoxyhexyl acrylate.

The result of the $^1H$-NMR analysis is shown in the following. $^1H$-NMR (500 MHz, $CDCl_3$): δ = 6.36 (d, J = 18.0 Hz, 1H), 6.09 (dd, J = 5.3 Hz, 8.8 Hz, 1H), 5.79 (d, J = 12.0 Hz, 1H), 4.13 (t, J = 7.0 Hz, 2H), 3.35 (t, J = 6.8 Hz, 2H), 3.31 (s, 3H), 1.66-1.56 (m, 4H), 1.37 (m, 4H).$^{13}C$-NMR (125 MHz, $CDCl_3$): δ = 166.4, 130.5, 128.6, 72.7, 64.6, 58.6, 29.6, 26.8, 25.8.

(G-2) Production of poly[6-methoxyhexyl acrylate] (see the following formula)

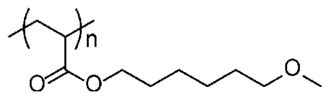

Synthesis was performed in the same manner as in (D-2) except that 5.05 g (28.0 mmol) of 6-methoxyhexyl acrylate, 25.1 g of 1,4-dioxane, and 5.03 mg (0.030 mmol) of azobisisobutyronitrile were used and the polymerization time was changed to 8 hours, and 3.75 g (percentage yield: 74%) of polymer was obtained.

The molecular weight was measured using a part of the thus-obtained polymer by using a method described later, and the number-average molecular weight (Mn) was found to be 29000 and the molecular weight distribution (Mw/Mn) was found to be 2.5. The glass transition temperature of the polymer was measured by using a method described later to be - 77.4°C, and $^1H$-NMR (500 MHz, $CDCl_3$) analysis identified the polymer as poly[6-methoxyhexyl acrylate]. $^1H$-NMR (500 MHz, $CDCl_3$): δ = 3.99 (br, 2H), 3.35 (t, J = 6.5 Hz, 2H), 3.31 (s, 3H), 2.24 (m, 1H), 1.58-1.35 (m, 10H).$^{13}C$-NMR (125 MHz, $CDCl_3$): δ = 174.7, 72.7, 64.6, 58.6, 41.5, 35.4, 29.6, 28.6, 25.9, 25.8.

(8) Examination of solubility of polymer material of general formula (1) in different solvents

To produce a coating solution, the solubility of each of PEt2A, PMe2MA, PEt2MA, PMC3A, PMC4A, PMC5A, and PMC6A prepared in (7) in different solvents was examined.

The results are shown in the table below.

It was found that the solubility of the polymer material of the general formula (1) in an ethanol/water system varies depending on the blend ratio of ethanol to water.

[Table 1]

| Ethanol/water system (25°C) | | | | | | | | |
|---|---|---|---|---|---|---|---|---|
| Ethanol/ water (g/g) | 100/0 | 90/10 | 80/20 | 70/30 | 50/50 | 35/65 | 30/70 | 20/80 |
| Solubility of PEt2A | soluble | soluble | Soluble | soluble | soluble | soluble | insoluble | insoluble |
| Solubility of PMe2MA | insoluble | insoluble | soluble | soluble | soluble | soluble | soluble | soluble |
| Solubility of PEt2MA | soluble | soluble | Soluble | soluble | soluble | insoluble | insoluble | insoluble |

[Table 2]

| Ethanol/water system (25°C) | | | | | | | | | |
|---|---|---|---|---|---|---|---|---|---|
| Ethanol/water system (g/g) | 100/0 | 80/20 | 70/30 | 60/40 | 50/50 | 40/60 | 35/65 | 30/70 | 20/80 |
| PMC3A | × | ○ | ○ | ○ | ○ | ○ | × | × | × |
| PMC4A | × | ○ | ○ | ○ | ○ | ○ | × | × | × |
| PMC5A | × | ○ | ○ | ○ | ○ | × | × | × | × |
| PMC6A | × | ○ | ○ | ○ | ○ | × | × | × | × |
| soluble: ○<br>insoluble: × | | | | | | | | | |

(Example 1)

A membrane-forming spinning dope was prepared by dissolving 17 parts by mass of polysulfone-based (manufactured by Solvay S.A., P-1700) and 4 parts by mass of polyvinylpyrrolidone (manufactured by BASF SE, K-90) in 79 parts by mass of dimethylacetamide (manufactured by Kishida Chemical Co., Ltd., reagent grade).

For a bore liquid, 60% by mass aqueous solution of dimethylacetamide was used.

The membrane-forming spinning dope and the bore liquid were discharged from a tube-in-orifice spinneret. The temperature of the membrane-forming spinning dope in discharging was 40°C. The membrane-forming spinning dope discharged was allowed to pass through a dropping portion under a hood into a coagulation bath containing water at 60°C, and soaked therein for coagulation. The spinning speed was 30 m/min.

After coagulation, washing with water and drying were performed to obtain a separation membrane in a hollow shape. The temperature for washing with water was 90°C, and the duration for washing with water was 180 seconds. The amount of discharge of each of the membrane-forming spinning dope and the bore liquid was adjusted so that the thickness of the membrane became 35 μm and the inner diameter became 185 μm after drying.

Hollow fiber separation membranes obtained were incorporated in a blood processing device and fixed, and thus a module with an effective area of 1.5 $m^2$ was set up. Then, 0.1 g of PEt2A (Mn: 11,600, Mw/Mn: 3.9) was dissolved in an aqueous solution (100 g) consisting of 35 g of ethanol/65 g of water to prepare a coating solution. The module set up was held vertically, and the coating solution was allowed to flow therethrough from the top at a flow rate of 100 mL/min to bring the coating solution into contact with the surface of the separation membranes.

The blood compatibility test was performed for the blood processing device obtained at this point, and the result showed that the LDH activity was 0.2.

After contact with the coating solution, the coating solution in the module was blown away with air at 0.1 KMpa, and the module was put in a vacuum dryer and vacuum-dried at 35°C for 15 hours, and γ-ray sterilization was performed at 25 Kgy in the atmosphere to obtain a blood processing device.

The blood compatibility test was performed for the blood processing device obtained, and the result showed that the LDH activity was 0.2 and the number of hollow fibers with residual blood was 0. It was revealed that the blood compatibility is hardly lowered even after being subjected to radiation sterilization in a dry state in the atmosphere.

The infrared ATR measurement was performed for the sample. Figure 1 shows the infrared absorption curve.

A peak corresponding to infrared absorption by the ester group (-O-C=O) (around 1735 $cm^{-1}$) derived from PEt2A was confirmed.

The ratio between the area of infrared absorption (around 1735 $cm^{-1}$), P1, and the area of infrared absorption (around 1595 $cm^{-1}$), P2, P1/P2, was 0.089.

The pyrolysis gas chromatography-mass spectrometry was performed for the sample.

Figure 3 shows the result. The result of the pyrolysis gas chromatography-mass spectrometry for PEt2A as a control is shown in Figure 2.

While the chromatogram peak derived from the pyrolysate of PEt2A was found around RT 7.9 min (Figure 2), a similar signature was found for the sample (Figure 3). From the searching result for the mass spectra (Figure 4), this peak was revealed to be derived from 2-(2-ethoxyethoxy)ethyl alcohol. Since 2-(2-ethoxyethoxy)ethyl alcohol is considered to be derived from hydrolysis of the pyrolysate of PEt2A (the side chain portion), the presence of PEt2A on the surface of the separation membrane of Example 1 was confirmed from the result.

The pyrolysis gas chromatography-mass spectrometry was performed for a separation membrane without a coating of PEt2A, which will be described later (Comparative Example 1), in the same manner, and no signature of a peak was found at RT 7.9 min.

The contact angle of the sample was measured.
The results are shown in the table below.
The contact angle was about 60°, and was not changed by repeated priming.

[Table 3]

| Number of primings | 1 | 2 | 3 | 4 | 5 |
|---|---|---|---|---|---|
| Contact angle ° | 58 | 57 | 56 | 57 | 57 |

The UFR (mL/Hr·mmHg) was measured for the sample to indicate UFR = 470 (mL/Hr·mmHg).

(Examples 2 to 6)

Hollow fiber separation membranes were formed in the same manner as in Example 1, which were incorporated in a blood processing device and fixed, and thus a module with an effective area of 1.5 $m^2$ was set up.
Then, a blood processing device was produced in the same manner as in Example 1 except that the PEt2A concentration and mixing ratio between water and the organic solvent (ethanol) in the coating solution were changed as shown in the table below, and the LDH activity, the number of hollow fibers with residual blood, and the infrared absorption peak ratio were measured.
The results are shown in Table 1.
Although the LDH activity was found to be slightly improved when the PEt2A concentration was increased, the difference was not significant.
When the solvent mixing ratio (ETOH/$H_2O$) is changed, on the other hand, as the quantity of the organic solvent increases, the peak ratio (P1/P2) tends to become smaller, i.e., the abundance of PEt2A on the surface of the separation membrane tends to decrease, and the LDH activity tends to increases, i.e., the blood compatibility tends to decrease. However, the LDH activity is in the range of those of commercially available products.

[Table 4]

| | PEt2A concentration (Wt%) | Solvent ratio ETOH/$H_2O$ | LDH activity | Number of hollow fibers with residual blood | Peak ratio (P1/P2) |
|---|---|---|---|---|---|
| Example-2 | 0.05 | 35/65 | 0.3 | 0 | 0.081 |
| Example-3 | 0.20 | 35/65 | 0.2 | 0 | 0.105 |
| Example-4 | 0.1 | 60/40 | 2.5 | 0 | 0.062 |
| Example-5 | 0.1 | 70/30 | 3.7 | 0 | 0.055 |
| Example-6 | 0.1 | 80/20 | 22 | 2 | 0.021 |

The samples of Examples 2 to 6 were analyzed through the pyrolysis gas chromatography-mass spectrometry in the same manner as in Example 1. The peak at RT 7.9 min as the peak derived from the pyrolysate of PEt2A was found for all of the samples, and the peak was revealed to be a peak derived from 2-(2-ethoxyethoxy)ethyl alcohol from the searching result for the mass spectra. From the result, the presence of PEt2A on the surface of the separation membrane was confirmed also in Examples 2 to 6.

(Examples 7 and 8)

Hollow fiber separation membranes were formed in the same manner in Example 1 except that, for the membrane-forming spinning dope, the quantity of polysulfone-based (manufactured by Solvay S.A., P-1700) and the quantity of polyvinylpyrrolidone (manufactured by BASF SE, K-90) with respect to 79 parts by mass of dimethylacetamide (manufactured by Kishida Chemical Co., Ltd., reagent grade) were changed as shown in the table below, and the hollow fiber separation membranes were incorporated in a blood processing device and coated with PEt2A, and the LDH activity

was measured.

The results are shown in the table below. Even when the composition of the membrane-forming dope was changed, the LDH activity was small and the blood compatibility was good.

[Table 5]

| | DMA concentra tion (Wt%) | PS concentra tion (Wt%) | PV concentra tion (Wt%) | LDH activity | Number of hollow fibers with residual blood | Peak ratio (P1/P2) |
|---|---|---|---|---|---|---|
| Example -7 | 79 | 19 | 2 | 1.5 | 0 | 0.082 |
| Example -8 | 79 | 15 | 6 | 0.4 | 0 | 0.095 |

(Example 9)

Hollow fiber separation membranes were formed in the same manner as in Example 1, which were incorporated in a blood processing device and fixed, and thus a module with an effective area of 1.5 $m^2$ was set up.

Then, 0.1 g of PMe2MA (Mn: 104,300, Mw/Mn: 4.6) was dissolved in an aqueous solution (100 g) consisting of 20 g of ethanol/ 80 g of water to prepare a coating solution. The module set up was held vertically, and the coating solution was allowed to flow therethrough from the top at a flow rate of 100 mL/min to bring the coating solution into contact with the surface of the separation membranes.

After contact with the coating solution, the coating solution in the module was blown away with air at 0.1 KMpa, and the module was put in a vacuum dryer and vacuum-dried at 35°C for 15 hours, and $\gamma$-ray sterilization was performed at 25 Kgy in the atmosphere to obtain a blood processing device.

The blood compatibility test was performed for the blood processing device obtained, and the result showed that the LDH activity was 1.7 and the number of hollow fibers with residual blood was 0.

The result of the ATR analysis showed that the P1/P2 ratio was 0.039.

The pyrolysis gas chromatography-mass spectrometry was performed for the sample.

While the chromatogram peak derived from the pyrolysate of PMe2MA is present around RT 12.7 min, a similar signature was found for the sample. From the searching result for the mass spectra (Figure 5), this peak was revealed to be derived from 2-(2-methoxyethoxy)ethyl methacrylate. Since 2-(2-methoxyethoxy)ethyl methacrylate is considered to be derived from hydrolysis of the pyrolysate of PMe2MA, the presence of PMe2MA on the surface of the separation membrane of Example 9 was confirmed from the result.

(Example 10)

Hollow fiber separation membranes were formed in the same manner as in Example 1, which were incorporated in a blood processing device and fixed, and thus a module with an effective area of 1.5 $m^2$ was set up.

Then, 0.1 g of PEt2MA (Mn: 142,500, Mw/Mn: 6.1) was dissolved in an aqueous solution (100 g) consisting of 40 g of ethanol/60 g of water to prepare a coating solution. The module set up was held vertically, and the coating solution was allowed to flow therethrough from the top at a flow rate of 100 mL/min to bring the coating solution into contact with the surface of the separation membranes.

After contact with the coating solution, the coating solution in the module was blown away with air at 0.1 KMpa, and the module was put in a vacuum dryer and vacuum-dried at 35°C for 15 hours, and $\gamma$-ray sterilization was performed at 25 Kgy in the atmosphere to obtain a blood processing device.

The blood compatibility test was performed for the blood processing device obtained, and the result showed that the LDH activity was 2.3 and the number of hollow fibers with residual blood was 0.

The result of the ATR analysis showed that the P1/P2 ratio was 0.039.

(Example 11)

A membrane-forming spinning dope was prepared by dissolving 17 parts by mass of polysulfone-based (manufactured by Solvay S.A., P-1700) and 4 parts by mass of polyvinylpyrrolidone (manufactured by BASF SE, K-90) in 79 parts by mass of dimethylacetamide (manufactured by Kishida Chemical Co., Ltd., reagent grade).

For a bore liquid, 60% by mass aqueous solution of dimethylacetamide was used.

The membrane-forming spinning dope and the bore liquid were discharged from a tube-in-orifice spinneret.

The temperature of the membrane-forming spinning dope in discharging was 40°C. The membrane-forming spinning dope discharged was allowed to pass through a dropping portion under a hood into a coagulation bath containing water at 60°C, and soaked therein for coagulation. The spinning speed was 30 m/min.

After coagulation, washing with water and drying were performed to obtain separation membranes in a hollow shape. The temperature for washing with water was 90°C, and the duration for washing with water was 180 seconds. The amount of discharge of each of the membrane-forming spinning dope and the bore liquid was adjusted so that the thickness of the membrane became 35 μm and the inner diameter became 185 μm after drying.

The hollow fiber separation membranes obtained were incorporated in a blood processing device and fixed, and thus a module with an effective area of 1.5 $m^2$ was set up. Then, 0.1 g of PMC3A (Mn: 31,000, Mw/Mn: 2.5) was dissolved in an aqueous solution (100 g) consisting of 40 g of ethanol/60 g of water to prepare a coating solution. The module set up was held vertically, and the coating solution was allowed to flow therethrough from the top at a flow rate of 100 mL/min to bring the coating solution into contact with the surface of the separation membranes.

After contact with the coating solution, the coating solution in the module was blown away with air at 0.1 KMpa, and the module was put in a vacuum dryer and vacuum-dried at 35°C for 15 hours.

The blood compatibility test (evaluation of lactate dehydrogenase (LDH) activity) was performed for the blood processing device obtained at this point, and the result showed that the LDH activity was 0.5.

This blood processing device was subjected to γ-ray sterilization at 25 Kgy in the atmosphere, and the blood compatibility test was performed for the resulting blood processing device, and the result showed that the LDH activity was 0.6 and the number of hollow fibers with residual blood was 0. It was revealed that the blood compatibility is hardly lowered even after being subjected to radiation sterilization in a dry state in the atmosphere.

The infrared ATR measurement was performed for the sample. Figure 6 shows the infrared absorption curve.

A peak corresponding to infrared absorption by the ester group (-O-C=O) (around 1735 $cm^{-1}$) derived from PMC3A was confirmed.

The ratio between the area of infrared absorption (around 1735 $cm^{-1}$), P1, and the area of infrared absorption (around (1595 $cm^{-1}$), P2, P1/P2, was 0.084.

The pyrolysis gas chromatography-mass spectrometry was performed for the sample.

Figure 8 shows the result. The result of the pyrolysis gas chromatography-mass spectrometry for single PMC3A polymer as a control is shown in Figure 7.

While the chromatogram peak derived from the pyrolysate of PMC3A was found around RT 3.2 min (Figure 7), a similar signature was found for the sample (Figure 8). From the searching result for the mass spectra (Figure 9), this peak was revealed to be derived from trimethylene glycol monomethyl ether. Since trimethylene glycol monomethyl ether is considered to be derived from hydrolysis of the pyrolysate of PMC3A (the side chain portion), the presence of PMC3A on the surface of the separation membrane of Example 11 was confirmed from the result.

The pyrolysis gas chromatography-mass spectrometry was performed for a separation membrane without a coating of PMC3A, which will be described later (Comparative Example 1), in the same manner, and no signature of a peak was found at RT 3.2 min.

The contact angle of the sample was measured.

The results are shown in the table below.

The contact angle was about 60°, and was not changed by repeated priming.

[Table 6]

| Number of primings | 1 | 2 | 3 | 4 | 5 |
|---|---|---|---|---|---|
| Contact angle ° | 61 | 60 | 60 | 59 | 60 |

The UFR (mL/Hr·mmHg) was measured for the sample to indicate UFR = 470 (mL/Hr·mmHg).

(Examples 12 to 16)

Hollow fiber separation membranes were formed in the same manner as in Example 11, which were incorporated in a blood processing device and fixed, and thus a module with an effective area of 1.5 $m^2$ was set up.

Then, a blood processing device was produced in the same manner as in Example 11 except that the PMC3A concentration, mixing ratio between water and the organic solvent (ethanol), or the type of the organic solvent in the coating solution was changed as shown in the table below, and the LDH activity, the number of hollow fibers with residual blood, and the infrared absorption peak ratio were measured.

The results are shown in the table below.

Even when the PMC3A concentration was increased, the LDH activity was not largely changed and a significant

difference was not found.

When the solvent mixing ratio (ETOH/$H_2O$) is changed, on the other hand, as the quantity of the organic solvent increases, the peak ratio (P1/P2) tends to become smaller, i.e., the abundance of PMC3A on the surface of the separation membrane tends to decrease, and the LDH activity tends to increases, i.e., the blood compatibility tends to decrease. However, the LDH activity is in the range of those of commercially available products .

[Table 7]

| | PMC3A concentration (Wt%) | Solvent ratio ETOH/H2O | LDH activity | Number of hollow fibers with residual blood | Peak ratio (P1/P2) |
|---|---|---|---|---|---|
| Example-11 | 0.10 | 40/60 | 0.6 | 0 | 0.084 |
| Example-12 | 0.05 | 40/60 | 1.2 | 0 | 0.076 |
| Example-13 | 0.20 | 40/60 | 0.7 | 0 | 0.082 |
| Example-14 | 0.10 | 60/40 | 3.5 | 0 | 0.051 |
| Example-15 | 0.10 | 80/20 | 25.0 | 1 | 0.019 |
| ETOH: Ethanol | | | | | |
| | PMC3A concentration (Wt%) | Solvent ratio MTOH/H2O | LDH activity | Number of hollow fibers with residual blood | Peak ratio (P1/P2) |
| Example-16 | 0.10 | 60/40 | 3.7 | 0 | 0.059 |
| MTOH: methanol | | | | | |

(Examples 17 and 18)

Hollow fiber separation membranes were formed in the same manner in Example 11 except that, for the membrane-forming spinning dope, the quantity of polysulfone-based (manufactured by Solvay S.A., P-1700) and the quantity of polyvinylpyrrolidone (manufactured by BASF SE, K-90) with respect to 79 parts by mass of dimethylacetamide (manufactured by Kishida Chemical Co., Ltd., reagent grade) were changed as shown in the table below, and the hollow fiber separation membranes were incorporated in a blood processing device and coated with PMC3A, and the LDH activity was measured.

The results are shown in the table below. Even when the composition of the membrane-forming dope was changed, the LDH activity was small and the blood compatibility was good.

[Table 8]

| | DMA concentration (Wt%) | PS concentration (Wt%) | PVP concentration (Wt%) | LDH activity | Number of hollow fibers with residual blood | Peak ratio (P1/P2) |
|---|---|---|---|---|---|---|
| Example-17 | 79 | 19 | 2 | 1.2 | 0 | 0.078 |
| Example-18 | 79 | 15 | 6 | 0.2 | 0 | 0.085 |

(Example 19)

Hollow fiber separation membranes were formed in the same manner as in Example 11, which were incorporated in a blood processing device and fixed, and thus a module with an effective area of 1.5 $m^2$ was set up.

Then, 0.1 g of PMC4A (poly[4-methoxybutyl acrylate]) (Mn: 29.000, Mw/Mn: 2.2) was dissolved in an aqueous

solution (100 g) consisting of 40 g of ethanol/ 60 g of water to prepare a coating solution. The module set up was held vertically, and the coating solution was allowed to flow therethrough from the top at a flow rate of 100 mL/min to bring the coating solution into contact with the surface of the separation membranes.

After contact with the coating solution, the coating solution in the module was blown away with air at 0.1 KMpa, and the module was put in a vacuum dryer and vacuum-dried at 35°C for 15 hours, and γ-ray sterilization was performed at 25 Kgy in the atmosphere to obtain a blood processing device.

The blood compatibility test was performed for the blood processing device obtained, and the result showed that the LDH activity was 1.1 and the number of hollow fibers with residual blood was 0.

The result of the ATR analysis showed that the P1/P2 ratio was 0.069.

(Example 20)

Hollow fiber separation membranes were formed in the same manner as in Example 11, which were incorporated in a blood processing device and fixed, and thus a module with an effective area of 1.5 $m^2$ was set up.

Then, 0.1 g of PMC5A (Mn: 50.000, Mw/Mn: 2.3) was dissolved in an aqueous solution (100 g) consisting of 45 g of ethanol/55 g of water to prepare a coating solution. The module set up was held vertically, and the coating solution was allowed to flow therethrough from the top at a flow rate of 100 mL/min to bring the coating solution into contact with the surface of the separation membranes.

After contact with the coating solution, the coating solution in the module was blown away with air at 0.1 KMpa, and the module was put in a vacuum dryer and vacuum-dried at 35°C for 15 hours, and γ-ray sterilization was performed at 25 Kgy in the atmosphere to obtain a blood processing device.

The blood compatibility test was performed for the blood processing device obtained, and the result showed that the LDH activity was 1.5 and the number of hollow fibers with residual blood was 0.

The result of the ATR analysis showed that the P1/P2 ratio was 0.071.

(Example 21)

Hollow fiber separation membranes were formed in the same manner as in Example 11, which were incorporated in a blood processing device and fixed, and thus a module with an effective area of 1.5 $m^2$ was set up.

Then, 0.1 g of PMC6A (Mn: 29.000, Mw/Mn: 2.5) was dissolved in an aqueous solution (100 g) consisting of 45 g of ethanol/55 g of water to prepare a coating solution. The module set up was held vertically, and the coating solution was allowed to flow therethrough from the top at a flow rate of 100 mL/min to bring the coating solution into contact with the surface of the separation membranes.

After contact with the coating solution, the coating solution in the module was blown away with air at 0.1 KMpa, and the module was put in a vacuum dryer and vacuum-dried at 35°C for 15 hours, and γ-ray sterilization was performed at 25 Kgy in the atmosphere to obtain a blood processing device.

The blood compatibility test was performed for the blood processing device obtained, and the result showed that the LDH activity was 1.9 and the number of hollow fibers with residual blood was 0.

The result of the ATR analysis showed that the P1/P2 ratio was 0.068.

(Comparative Example 1)

A module with an effective area of 1.5 $m^2$ was set up in the same manner as in Example 1 and Example 11 except that the separation membrane was not contacted with a coating solution. The blood compatibility test was performed for the module, and the result showed that the LDH activity was 100 and the number of hollow fibers with residual blood was 6. The LDH activity before the radiation sterilization was 10, which indicates that the degradation of the blood compatibility is larger than that in Example 1.

The infrared ATR measurement was performed for the sample. However, the infrared absorption peak (around 1735 $cm^{-1}$) was not found in the absorption curve.

The pyrolysis gas chromatography-mass spectrometry was performed for the sample. However, neither 2-(2-ethoxyethoxy)ethyl alcohol nor PMC3A (poly[3-methoxypropyl acrylate]) was found.

The contact angle was measured in the same manner as in Example 1. The results are shown in the table below. The contact angle was about 70°, and was not changed by repeated priming.

[Table 9]

| Number of primings | 1 | 2 | 3 | 4 | 5 |
|---|---|---|---|---|---|
| Contact angle ° | 69 | 72 | 70 | 70 | 71 |

Summary of the above results is shown in the following table.

[Table 10]

| | Polymer | Polymer concentration (Wt%) | Solvent ratio (ETOH/$H_2O$) | LDH activity | Number of hollow fibers with residual blood | Peak ratio (P1/P2) |
|---|---|---|---|---|---|---|
| Example-1 | PEt2A | 0.1 | 35/65 | 0.2 | 0 | 0.089 |
| Example-9 | PMe2MA | 0.1 | 20/80 | 1.7 | 0 | 0.039 |
| Example-10 | PEt2MA | 0.1 | 40/60 | 2.3 | 0 | 0.039 |
| Example-11 | PMC3A | 0.1 | 40/60 | 0.6 | 0 | 0.084 |
| Example-19 | PMC4A | 0.1 | 40/60 | 1.1 | 0 | 0.069 |
| Example-20 | PMC5A | 0.1 | 45/55 | 1.5 | 0 | 0.071 |
| Example-21 | PMC6A | 0.1 | 45/55 | 1.9 | 0 | 0.068 |
| Comparative Example -1 | - | - | - | 100 | 6 | ≤0.001 |
| LDH activities and peak ratios are each a value after sterilization (25 Kgy). | | | | | | |

(Comparative Example 2)

Separation membranes were formed in the same manner as in Example 1 except that polyvinylpyrrolidone was not added to the membrane-forming spinning dope, and the separation membranes were incorporated in a blood processing device and fixed in the same manner as in Example 1, and coated with PEt2A. For the resultant, the blood compatibility test was performed, and the result showed that the LDH activity was 25 and the number of hollow fibers with residual blood was 3.

The contact angle was measured for the sample. The results are shown in the table below. The contact angle was changed to a contact angle indicative of hydrophobicity by repeated priming. This is presumably because PEt2A on the surface of the separation membrane was fixed in an unstable manner.

[Table 11]

| Number of primings | 1 | 2 | 3 | 4 | 5 |
|---|---|---|---|---|---|
| Contact angle ° | 60 | 62 | 66 | 70 | 72 |

(Comparative Example 3)

Separation membranes were formed in the same manner as in Example 11 except that polyvinylpyrrolidone was not added to the membrane-forming spinning dope, and the separation membranes were incorporated in a blood processing device and fixed in the same manner as in Example 11, and coated with PMC3A. For the resultant, the blood compatibility test was performed, and the result showed that the LDH activity was 35 and the number of hollow fibers with residual blood was 1.

The contact angle was measured for the sample, and the contact angle was found to be changed to a contact angle indicative of hydrophobicity by repeated priming. This is presumably because the adhesion strength between PMC3A and the separation membrane (single polysulfone-based) was insufficient, and the state of the PMC3A coating layer present on the surface of the separation membrane was unstable.

(Comparative Example 4)

The blood compatibility test was performed for the commercially available product CX-21U (manufactured by TORAY INDUSTRIES, INC.), which is not included in the present invention, in the same manner to measure the LDH activity and the number of hollow fibers with residual blood, and the result showed that the LDH activity was 66.2 and the number of hollow fibers with residual blood was 4.

The generation of residual blood suggests poor blood compatibility.

Here, hollow fibers without residual blood had been used for the LDH activity measurement.

<Protein attachment evaluation test>

(Example 22)

Hollow fiber separation membranes were formed in the same manner as in Example 11, and both ends of the separation membranes sampled were processed with epoxy resin (Bond Quick Set, Konishi Co., Ltd.) so that the effective length was 15 cm and the area of the inner surface of the membranes was $5 \times 10^{-3}$ $m^2$ to produce two hollow fiber-type blood processing devices.

The hollow fiber-type blood processing devices were held vertically, and 20 mL of PMC3A coating solution (a solution obtained by dissolving 0.1 g of PMC3A (Mn: 31,000, Mw/Mn: 2.5) in an aqueous solution (100 g) consisting of 40 g of ethanol/60 g of water) prepared in the same manner as in Example 11 or PEt2A coating solution (a solution obtained by dissolving 0.1 g of PEt2A (Mn: 11,600, Mw/Mn: 3.9) in an aqueous solution (100 g) consisting of 35 g of ethanol/65 g of water) prepared in the same manner as in Example 1 was allowed to flow therethrough from the top of each hollow fiber-type blood processing device at a flow rate of 1 mL/min to bring the coating solution into contact with the surface of the separation membranes. After contact with the coating solution, the coating solution in each hollow fiber-type blood processing device was blown away with air at 0.1 KMpa, and the hollow fiber-type blood processing devices were put in a vacuum dryer and vacuum-dried at 35°C for 15 hours.

Thereafter, γ-ray sterilization was performed for the hollow fiber-type blood processing devices at 25 Kgy in the atmosphere, and the blood compatibility evaluation with inflammatory model blood in (6-2) was performed for the resulting hollow fiber-type blood processing devices.

Further, the same evaluation was performed by using the blood of a heathy individual in place of the model blood.

The results are shown in the table below, and a photograph of the surface state of each hollow fiber separation membrane after the blood compatibility evaluation with inflammatory model blood is shown in each of Figures 10 and 11.

(Comparative Example 5)

Hollow fiber separation membranes were formed in the same manner as in Example 22, and both ends of the separation membranes sampled were processed with epoxy resin (Bond Quick Set, Konishi Co., Ltd.) so that the effective length was 15 cm and the area of the inner surface of the membranes was $5 \times 10^{-3}$ $m^2$ to produce a hollow fiber-type blood processing device.

In 7.2 liter of pure water, 5 g of sodium pyrosulfite and 1.75 g of sodium carbonate were mixed, and the resultant was stirred for 1 hour to prepare an antioxidative solution. The hollow fiber-type blood processing device was filled with the antioxidative solution prepared, and sealed with a sealing plug, and the resultant was subjected to γ-ray sterilization at 25 Kgy in the atmosphere. The protein attachment tests with inflammatory model blood and blood from a healthy individual were performed for the resulting hollow fiber-type blood processing device in the same manner as in Example 22.

The results are shown in the table below, and a photograph of the surface state of the hollow fiber separation membrane after the blood compatibility evaluation with inflammatory model blood is shown in Figure 12.

In addition, the blood compatibility test (evaluation of lactate dehydrogenase (LDH) activity) was performed for this blood processing device, and the result showed that the LDH activity was 10.5.

[Table 12]

| | Polymer | Amount of attachment of proteins on inner surface of membrane (μg/ml) | |
|---|---|---|---|
| | | Normal blood | Inflammatory blood |
| Example22 | PMC3A | 57 | 353 |
| | PEt2A | 34 | 171 |
| Comparative Example 5 | - | 379 | 928 |

For both of the hollow fiber-type blood processing devices of Example 22, the amount of attachment of proteins was smaller than that in Comparative Example 5 both when inflammatory model blood was used and when blood from a healthy individual was used, and thus it is expected, for example, that generation of residual blood or the like in treatment is less frequent when any of the hollow fiber-type blood processing devices of Example 22 is used for dialysis treatment or the like.

When the surface condition of the hollow fiber separation membrane after the blood compatibility evaluation

with inflammatory model blood was observed, no noticeable attached substance was found for both of the cases with PMC3A and PEt2A in Example 22 (Figures 10 and 11), and attachment of fibrin was found on the surface for Comparative Example 5 (Figure 12).

Industrial Applicability

The separation membrane for blood processing and the blood processing device including the membrane, each according to the present invention, exhibit very good blood compatibility even after being subjected to radiation sterilization in a dry state in the atmosphere, and are further expected to have reduced degradation of the blood compatibility even after a long-term use, and thus can be suitably used for extracorporeal circulation therapies including hemodialysis, hemofiltration, hemodiafiltration, blood fractionation, oxygenation, and plasmapheresis.

The present application is based on a Japanese patent application (Japanese Patent Application No. 2015-125420) filed with the Japan Patent Office on June 23, 2015, and a Japanese patent application (Japanese Patent Application No. 2016-076397) filed with the Japan Patent Office on April 6, 2016, and the contents of them are incorporated herein by reference.

## Claims

1. A separation membrane for blood processing comprising:

    a separation membrane containing polysulfone-based polymer and polyvinylpyrrolidone; and
    a layer coated on at least a part of the surface of the separation membrane and containing a polymer material having a structure represented by following general formula (1):

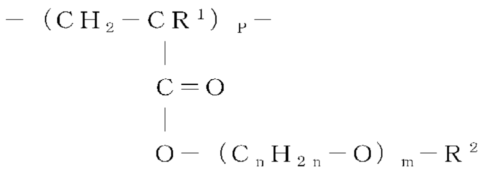

(1)

wherein $R^1$ is a hydrogen atom or a methyl group; $R^2$ is a methyl group or an ethyl group; n is from 2 to 6 and m is from 1 to 3; P denotes a number of repetition; and a plurality of each of $R^1$, $R^2$, n, and m present in one molecule may be the same or different.

2. The separation membrane for blood processing according to claim 1, wherein a number-average molecular weight of the polymer material having the structure represented by the general formula (1) is from 8,000 to 300,000.

3. The separation membrane for blood processing according to claim 1 or 2, wherein, in an infrared absorption curve obtained in attenuated total reflection-infrared spectroscopy (ATR-IR) for the surface of the separation membrane, a ratio of a peak strength of an infrared absorption peak around 1735 $cm^{-1}$, P1, to a peak strength of an infrared absorption peak at 1595 $cm^{-1}$, P2, P1/P2, is 0.015 or higher.

4. The separation membrane for blood processing according to any one of claims 1 to 3, wherein, in the general formula (1), $R^1$ is a hydrogen atom, $R^2$ is an ethyl group, n is 2, and m is 2.

5. The separation membrane for blood processing according to any one of claims 1 to 3, wherein, in the general formula (1), $R^1$ is a methyl group, $R^2$ is a methyl group, n is 2, and m is 2.

6. The separation membrane for blood processing according to any one of claims 1 to 3, wherein, in the general formula (1), $R^1$ is a methyl group, $R^2$ is an ethyl group, n is 2, and m is 2.

7. The separation membrane for blood processing according to any one of claims 1 to 3, wherein, in the general formula (1), $R^1$ is a hydrogen atom, $R^2$ is a methyl group, n is 3, and m is 1.

8. The separation membrane for blood processing according to any one of claims 1 to 3, wherein, in the general formula (1), $R^1$ is a hydrogen atom, $R^2$ is a methyl group, n is 4, and m is 1.

**9.** The separation membrane for blood processing according to any one of claims 1 to 3, wherein, in the general formula (1), $R^1$ is a hydrogen atom, $R^2$ is a methyl group, n is 5, and m is 1.

**10.** The separation membrane for blood processing according to any one of claims 1 to 3, wherein, in the general formula (1), $R^1$ is a hydrogen atom, $R^2$ is a methyl group, n is 6, and m is 1.

**11.** A blood processing device comprising the separation membrane for blood processing according to any one of claims 1 to 10.

**12.** A method for producing a separation membrane for blood processing, the method comprising:

a step of forming a separation membrane containing polysulfone-based polymer and polyvinylpyrrolidone; and
a step of coating at least a part of the surface of the separation membrane with a coating solution containing a polymer material having a structure represented by the general formula (1).

**13.** The method for producing a separation membrane for blood processing according to claim 12, wherein the coating solution contains water and an organic solvent, and the organic solvent is ethanol, methanol, or a mixture thereof.

**14.** The method for producing a separation membrane for blood processing according to claim 12 or 13, wherein, in the step of forming the separation membrane,
the separation membrane is formed by using a membrane-forming dope containing polysulfone-based polymer and polyvinylpyrrolidone, and a ratio of polyvinylpyrrolidone to polysulfone-based polymer (polyvinylpyrrolidone/polysulfone-based polymer) in the membrane-forming dope is 27% by mass or less.

**15.** A method for producing the blood processing device according to claim 11, the method comprising:

a step of forming a separation membrane containing polysulfone-based polymer and polyvinylpyrrolidone;
a step of potting to seal an inner space of the separation membrane from an outer space; and
a step of coating the surface of the separation membrane and the surface of the potting with a coating solution containing a polymer material having the structure represented by the general formula (1), wherein
the steps are performed in the order presented.

Figure 1

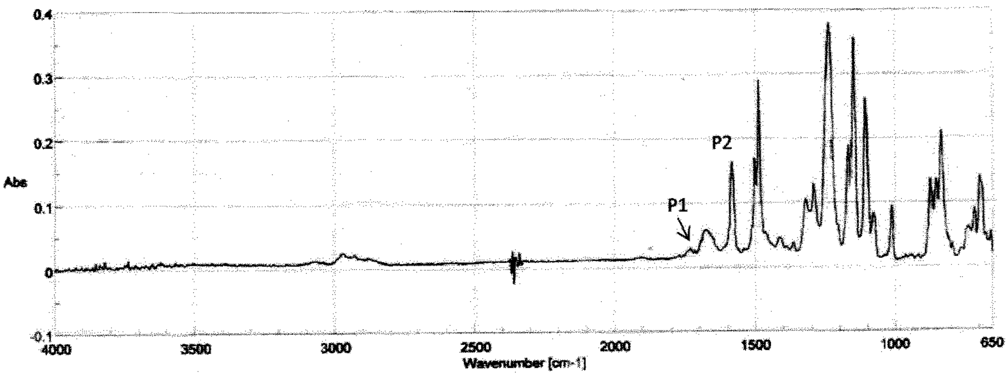


Figure 2

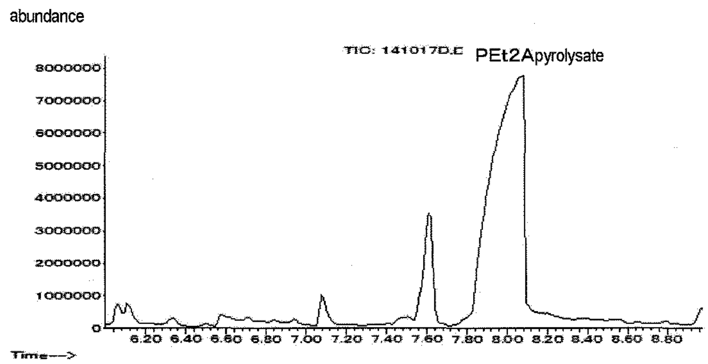


Figure 3

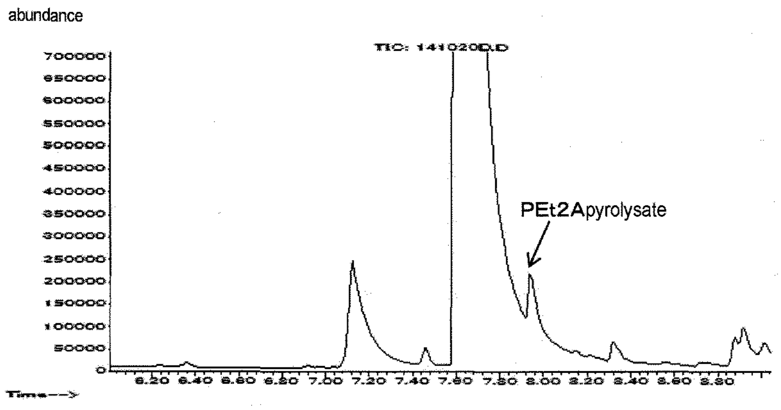

Figure 4

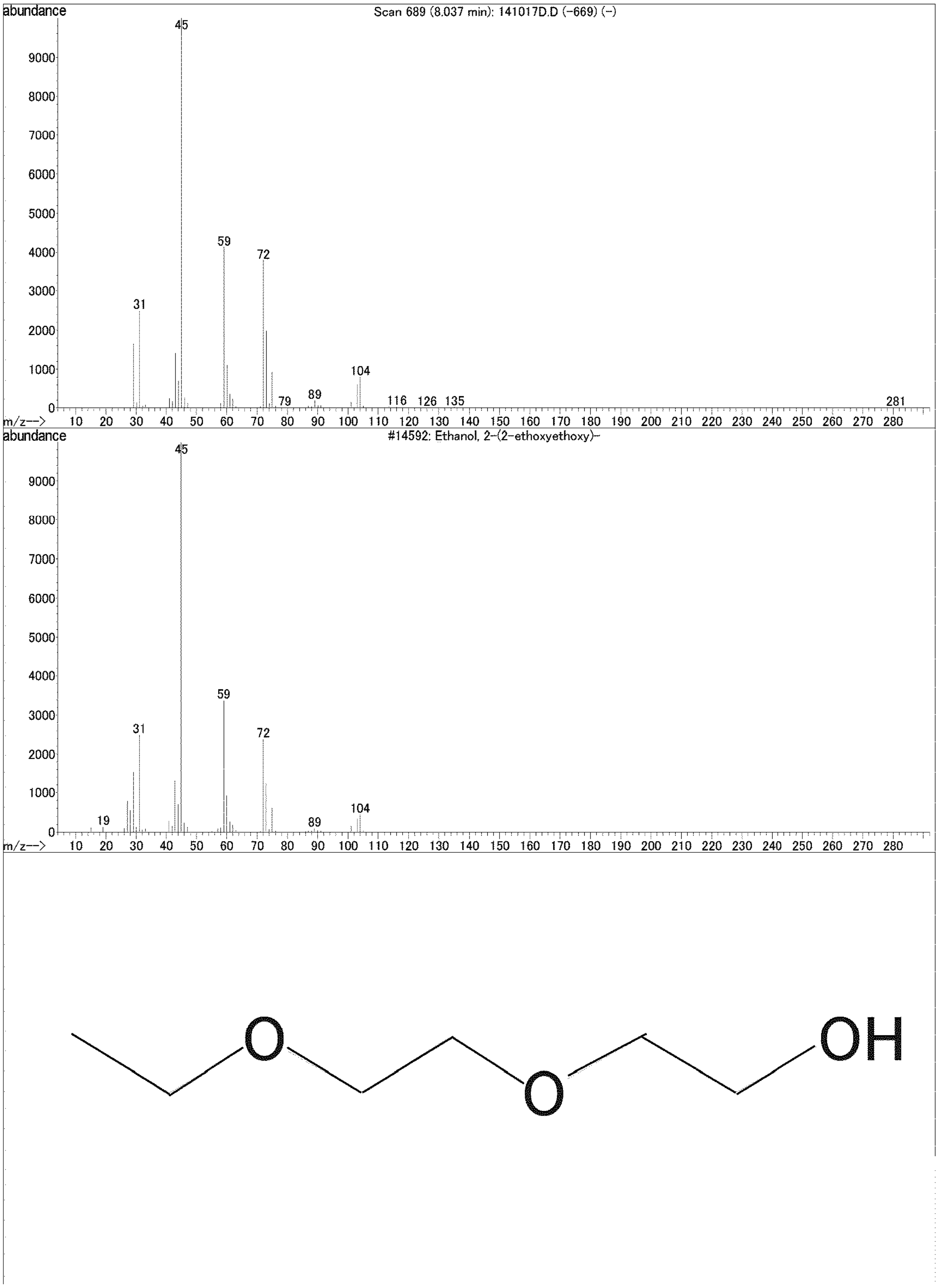
abundance
Scan 689 (8.037 min): 141017D.D (-669) (-)
45
59
72
31
104
79
89
116
126
135
281
m/z-->
abundance
#14592: Ethanol, 2-(2-ethoxyethoxy)-
45
59
31
72
19
89
104
m/z-->

Figure 5

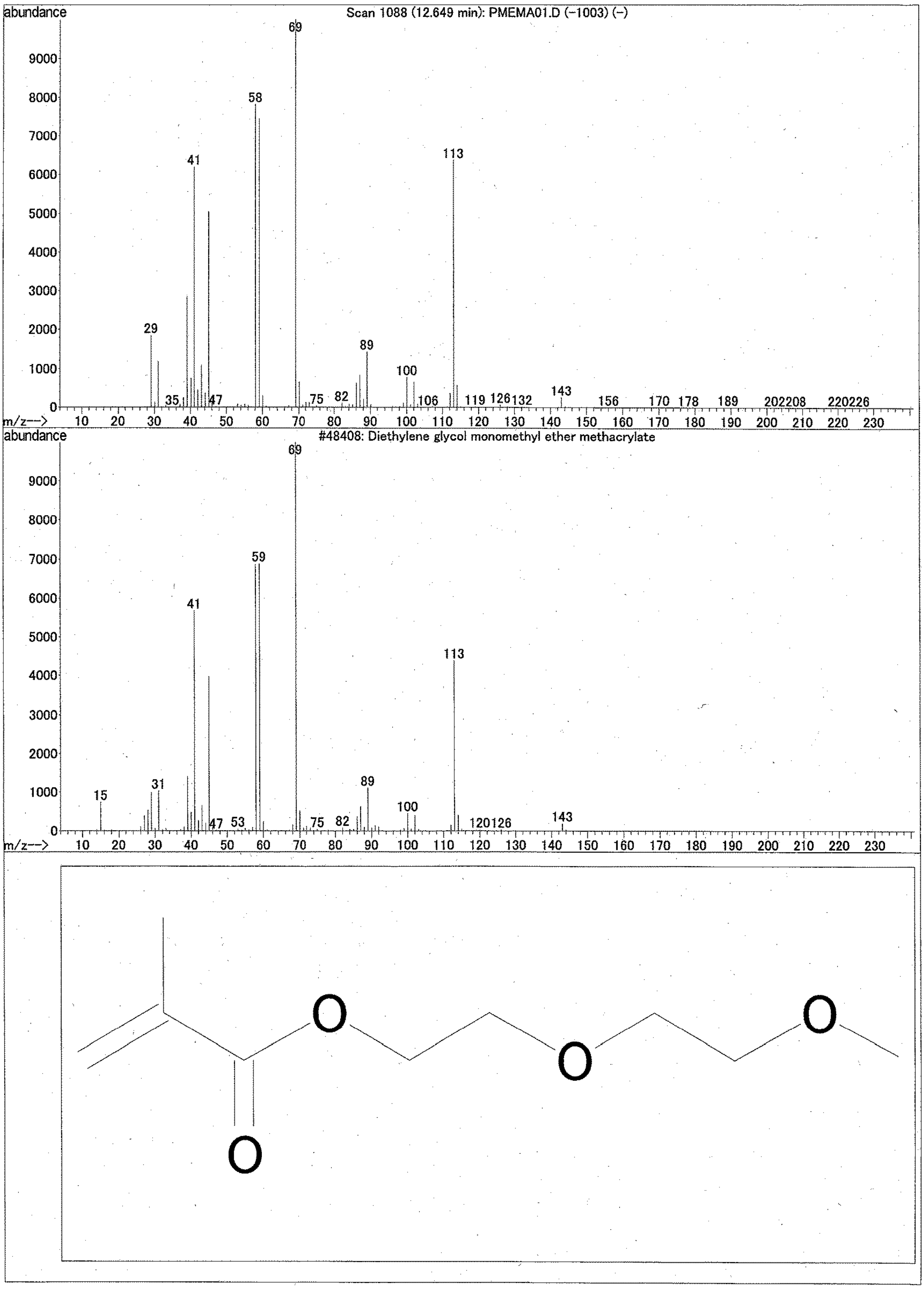
abundance
Scan 1088 (12.649 min): PMEMA01.D (-1003) (-)
9000
8000
7000
6000
5000
4000
3000
2000
1000
0
69
58
41
113
29
89
100
35
47
75
82
106
119
126
132
143
156
170
178
189
202
208
220
226
m/z-->
10 20 30 40 50 60 70 80 90 100 110 120 130 140 150 160 170 180 190 200 210 220 230
abundance
#48408: Diethylene glycol monomethyl ether methacrylate
9000
8000
7000
6000
5000
4000
3000
2000
1000
0
69
59
41
113
15
31
89
100
47
53
75
82
120
126
143
m/z-->
10 20 30 40 50 60 70 80 90 100 110 120 130 140 150 160 170 180 190 200 210 220 230

Figure 6

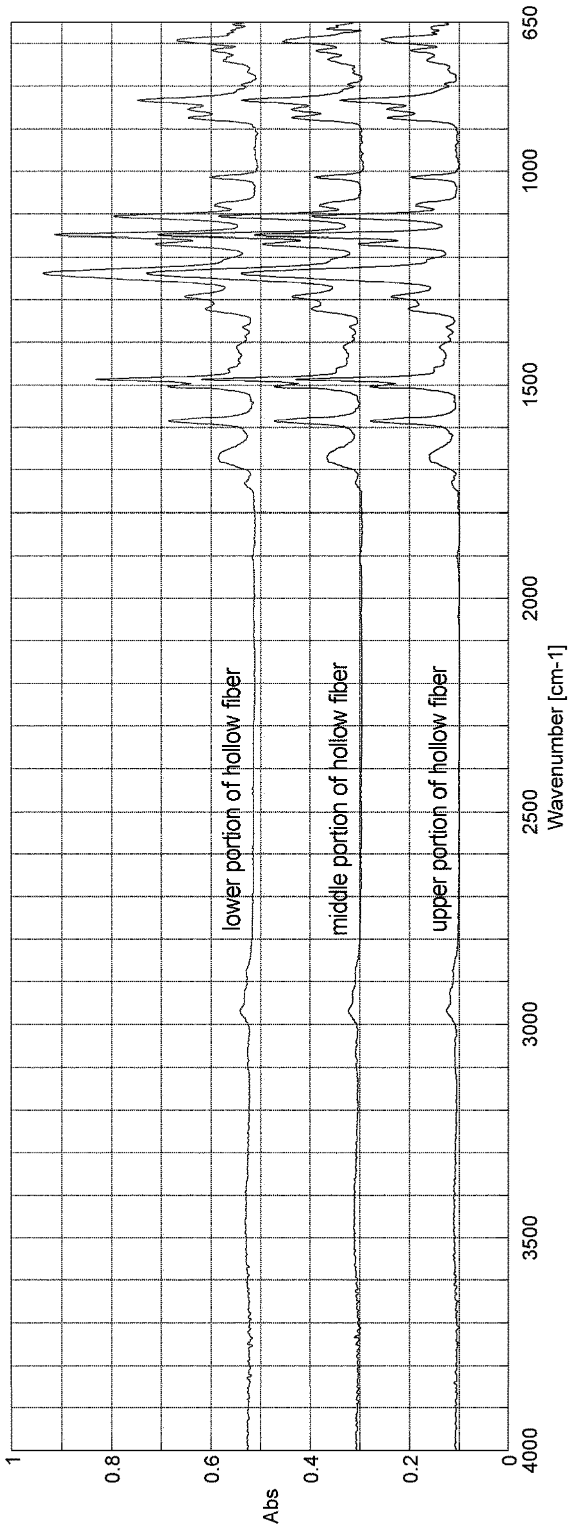
lower portion of hollow fiber
middle portion of hollow fiber
upper portion of hollow fiber
Abs
Wavenumber [cm-1]
650
1000
1500
2000
2500
3000
3500
4000
1
0.8
0.6
0.4
0.2
0

Figure 7

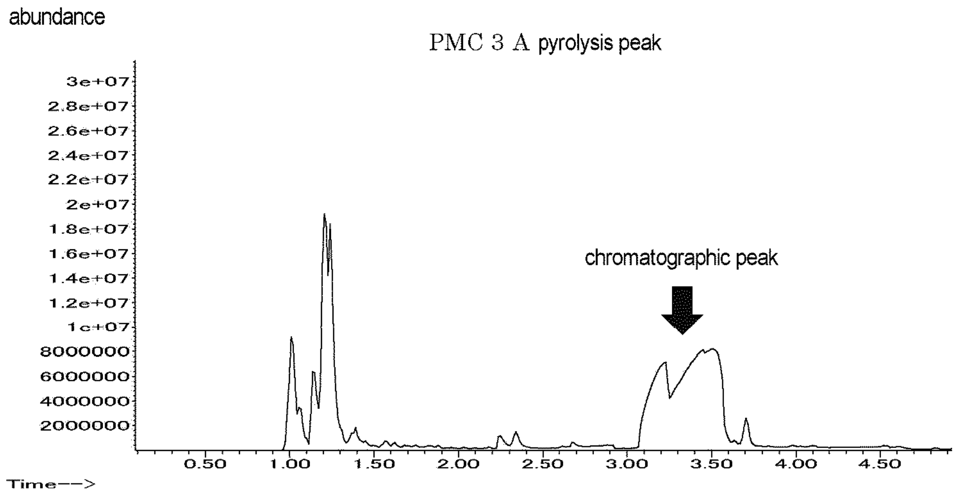


Figure 8

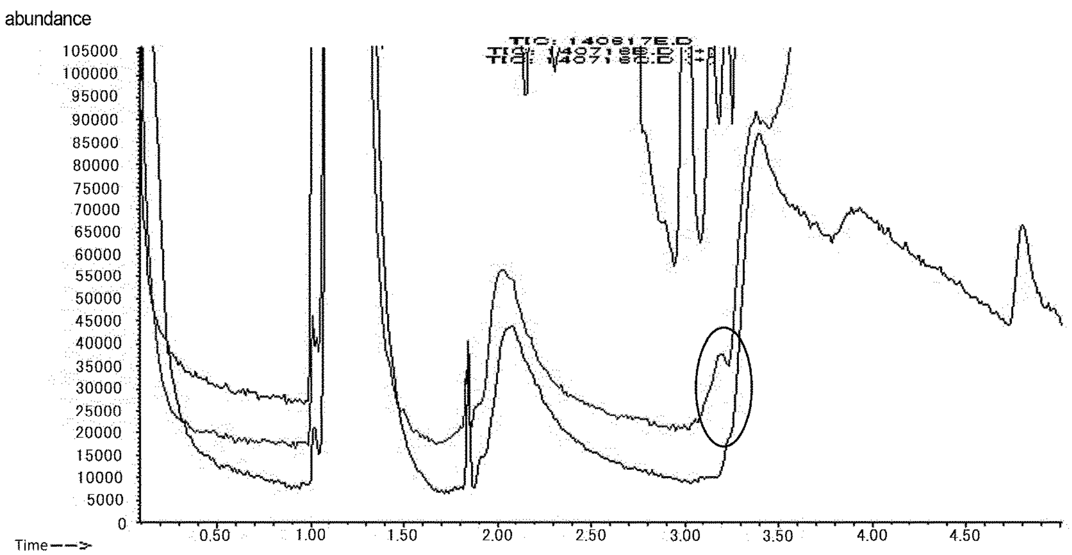

Figure 9

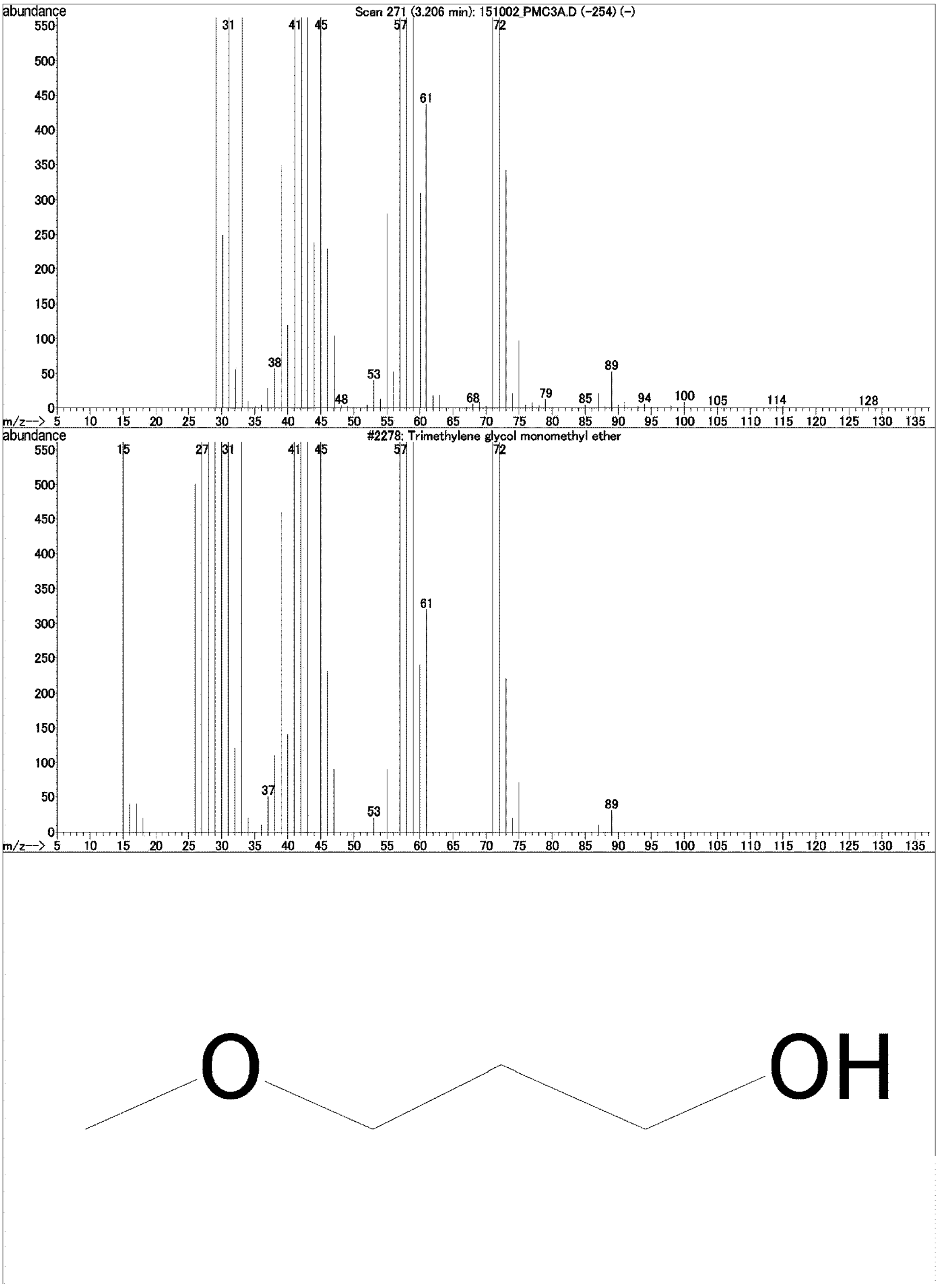
abundance
Scan 271 (3.206 min): 151002_PMC3A.D (-254) (-)
m/z-->
abundance
#2278: Trimethylene glycol monomethyl ether
m/z-->

Figure 10

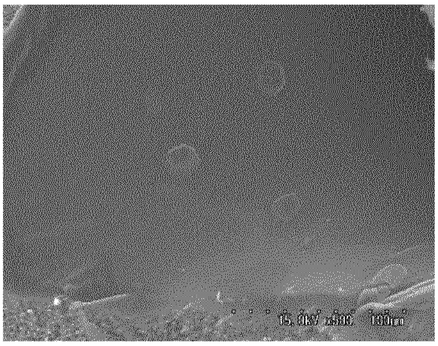

Figure 11

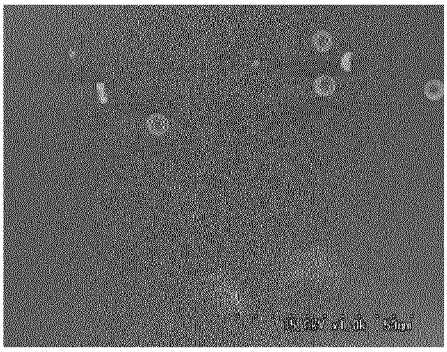

Figure 12

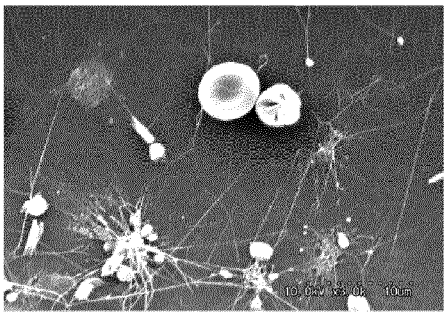

**INTERNATIONAL SEARCH REPORT**

International application No. PCT/JP2016/068571

A. CLASSIFICATION OF SUBJECT MATTER
*B01D71/68*(2006.01)i, *A61K35/14*(2015.01)i, *A61M1/18*(2006.01)i, *A61P7/08*(2006.01)i, *B01D69/10*(2006.01)i, *B01D69/12*(2006.01)i, *B01D71/44*(2006.01)i, *C08J7/04*(2006.01)i, *C08L39/06*(2006.01)i, *C08L81/06*(2006.01)i

According to International Patent Classification (IPC) or to both national classification and IPC

B. FIELDS SEARCHED

Minimum documentation searched (classification system followed by classification symbols)
B01D71/68, A61K35/14, A61M1/18, A61P7/08, B01D69/10, B01D69/12, B01D71/44, C08J7/04, C08L39/06, C08L81/06

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 4888559 B2 (Toray Industries, Inc.), 29 February 2012 (29.02.2012), paragraphs [0019] to [0020], [0070], [0098] & US 2011/0017654 A1 paragraphs [0041] to [0042], [0098], [0134] & US 2014/0061121 A1 & WO 2009/123088 A1 & EP 2286902 A1 & CN 102015081 A | 1-15 |
| Y | JP 4746984 B2 (Japan Science and Technology Agency), 10 August 2011 (10.08.2011), claim 1; page 7, lines 1 to 35 & WO 2004/087228 A1 | 1-15 |

☒ Further documents are listed in the continuation of Box C.

☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 14 September 2016 (14.09.16) | 27 September 2016 (27.09.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2016/068571

C (Continuation). DOCUMENTS CONSIDERED TO BE RELEVANT

| Category* | Citation of document, with indication, where appropriate, of the relevant passages | Relevant to claim No. |
|---|---|---|
| Y | JP 61-238306 A (Toray Industries, Inc.), 23 October 1986 (23.10.1986), claim 2 (Family: none) | 1-15 |
| Y | JP 5-262656 A (Terumo Corp.), 12 October 1993 (12.10.1993), paragraphs [0026], [0028] to [0029] (Family: none) | 1-15 |
| P,X | JP 2016-77570 A (Asahi Kasei Medical Co., Ltd., Yamagata University), 16 May 2016 (16.05.2016), claims 1 to 6 (Family: none) | 1-3,11-15 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4338223 A
- JP 7194949 A
- WO 2006016575 A
- JP 3908839 B
- JP 2015136383 A
- JP 4746984 B
- JP 2015125420 A
- JP 2016076397 A


### Non-patent literature cited in the description


- **N. YASUDA et al.** *J. Surg. Research,* 2012, vol. 176